# EUROPEAN PATENT SPECIFICATION

(11) **EP 2 208 072 B1**
(45) Date of publication and mention of the grant of the patent: **01.07.2015**
(21) Application number: 08842854.5
(22) Date of filing: 22.10.2008
(51) Int. Cl.: G01N 33/68, G01N 33/49, G01N 33/574

(54) **METHODS OF PROGNOSIS**
PROGNOSEVERFAHREN
PROCÉDÉS DE PRONOSTIC

(30) Priority: 22.10.2007 AU 2007905761
(43) Date of publication of application: 21.07.2010
(73) Proprietor: ST VINCENT'S HOSPITAL SYDNEY LIMITED, Darlinghurst, NSW 2010 (AU)
(72) Inventor: BREIT, Samuel, Norbert, Gordon, NSW 2072 (AU); BROWN, David, Alexander, Lane Cove, NSW 2066 (AU); GRÖNBERG, Henrik, 903 39 Umeå (SE)
(74) Representative: Harding, Charles Thomas
(86) International application number: PCT/AU2008/001554
(87) International publication number: WO 2009/052557

(56) References cited:
- WO-A1-01/81928
- WO-A2-02/059373
- WO-A2-03/093794
- BROWN DAVID A ET AL: "MIC-1 serum level and genotype: associations with progress and prognosis of colorectal carcinoma.", CLINICAL CANCER RESEARCH : AN OFFICIAL JOURNAL OF THE AMERICAN ASSOCIATION FOR CANCER RESEARCH JUL 2003 LNKD- PUBMED:12855642, vol. 9, no. 7, July 2003 (2003-07), pages 2642-2650, XP002613961, ISSN: 1078-0432
- BROWN DA ET AL.: 'Measurement of Serum Levels of Macrophage Inhibitory Cytokine I Combined with Prostate-Specific Antigen Improves Prostate Cancer Diagnosis' CLINICAL CANCER RESEARCH vol. 12, no. 1, 2006, pages 89 - 96, XP008132679
- BAUSKIN AR ET AL.: 'Role of Macrophage Inhibitory Cytokine-1 in Tumorigenesis and Diagnosis of Cancer' CANCER RESEARCH vol. 66, no. 10, 2006, pages 4983 - 4986, XP008132686
- WELSH JB ET AL.: 'Large-scale delineation of secreted protein biomarker overexpressed in cancer tissue and serum' PROC. NATL. ACAD. SCI. USA vol. 100, no. 6, 2003, pages 3410 - 3415, XP002993660
- NAKAMURA T ET AL.: 'Quantitative analysis of macrophage inhibitory cytokine-1 (MIC-1) gene expression in human prostatic tissues' BRITISH JOURNAL OF CANCER vol. 88, no. 7, 2003, pages 1101 - 1104, XP008132684
- RASIAH KK ET AL.: 'Aberrant Neuropeptide Y and Macrophage Inhibitory Cytokine-1 Expression Are Early Events in Prostate Cancer Development and Are Associated with Poor Prognosis' CANCER EPIDEMIOLOGY, BIOMARKERS & PREVENTION vol. 15, no. 4, 2006, pages 711 - 716, XP008132680

## Description

### FIELD OF THE INVENTION

The invention relates to the field of medical prognostics. In particular, the invention relates to methods for predicting prostate cancer progression and overall survival prognosis in a subject involving the detection of elevated amounts of macrophage inhibitory cytokine-1 (MIC-1) in a test body sample such as serum.

### BACKGROUND OF THE INVENTION

MIC-1 is a divergent member of the TGF-β superfamily first cloned on the basis of increased mRNA expression associated with macrophage activation¹. While MIC-1 is not expressed in resting macrophages, stimulation of macrophages by a number of biological mediators including tumour necrosis factor (TNF)-α, interleukin-1 (IL-1) and macrophage-colony stimulating factor (M-CSF) induce MIC-1 expression. Because of its induction by many pro-inflammatory cytokines, but failure of direct induction by lipopolysaccharide and interferon-γ (IFN-γ), it has been hypothesised that MIC-1 may be an autocrine down-regulator of macrophage activation¹.

MIC-1 can be expressed in several tissues³⁻⁶. Northern blots of human tissues indicate the presence of small amounts of MIC-1 mRNA in the kidney, pancreas and prostate, and large amounts in the placenta^{3,5}. Serum MIC-1 levels have been shown to increase with age in normal, apparently healthy subjects⁸ . MIC-1 overexpression has been associated with cancer, particularly prostate cancer⁷, and high serum concentrations of MIC-1 are associated with the presence of metastatic disease^{7,8}. MIC-1 has also been detected by immunohistochemistry in biopsies of breast, colon and prostate cancers⁶. However, MIC-1 is not detectable within normal epithelial cells of these organs⁶ . This, along with induction of MIC-1 expression by p53 and data suggesting that MIC-1 is able to induce apoptosis of some epithelial tumour cells lines⁹⁻¹¹, indicates a role for MIC-1 in epithelial neoplasms.

Abnormal levels of MIC-1 in a sample have been used in methods for diagnosing risk of miscarriage and/or premature birth, foetal abnormalities, cancer and inflammatory disease ³⁵.

It is known that there is an increase is the serum level of MIC-1 in some patient groups compared to normal individuals²⁹.

Prostate cancer is frequently diagnosed by an increased concentration of prostate-specific antigen (PSA) in serum when the prostate cancer is localised to the prostate gland, although there is currently some concern about the accuracy of this test. Additionally, managing the treatment of males newly diagnosed with localised prostate cancer remains a major clinical challenge, as a high proportion of subjects with untreated localised prostate cancer have an excellent prognosis as prostate cancer is usually non-fatal and frequently symptomless, whilst active treatment is associated with a serious impact on lifestyle (for example, loss of urinary control and impotence) and morbidity¹².

Presently, methods of safely discriminating between prostate cancers that will follow a benign course, from those that have a poor prognosis, wherein radical therapy may be beneficial, are inadequate. The "Gleason sum" (calculated out of 10) is one indicator that is presently used for prostate cancer severity: a tumour with a lower Gleason sum has tissue that is closer to normal histologically, and is less likely to be aggressive; whilst a tumour with a higher Gleason sum is more likely to be an aggressive tumour. However, this technique requires a biopsy of the prostate and histological analysis, and is accordingly an invasive and expensive technique that requires time consuming expert analysis.

Malignant tumours are classified using the tumour-node-metastasis (TNM) classification system developed and maintained by the International Union Against Cancer (UICC) to achieve consensus on one globally recognised standard for classifying the extent of spread of cancer.³⁴ TNM stage (I-IV) is an important factor used for understanding prostate cancer severity. The TNM system evaluates the size of the tumour (T score), the extent of lymph node involvement (N score), and any metastasis (M score), as well as using grading based on cellular morphology from which the Gleason sum is derived. Briefly, the T score is graded from 0 (no tumour) to 4; the N score is graded from 0 (no node spread) to 3; and the M score is graded 0 (no distant metastasis) to 1 (distant metastasis). A grade of "X" is given for any parameter that cannot be assessed. TNM Stage I prostate cancer has a score of T1, N0 and M0 and a Gleason sum of 4 or below, and is cancer that is found incidentally in a small part of the sample, usually because prostate tissue was removed for other reasons; the cells closely resemble normal cells and the gland feels normal to the examining finger. TNM Stage II prostate cancer has a score of T1-T2, N0 and M0 and a Gleason sum of 5 or more, and more of the prostate is involved and a lump can be felt within the gland. TNM Stage III prostate cancer has a score of T3, N0, M0 and any Gleason sum score, the tumour has spread through the prostatic capsule and the lump can be felt on the surface of the gland. TNM Stage IV prostate cancer has a score of T4, any N, any M and any Gleason sum, or any T, any Gleason Sum and either N1 and/or M1, and the tumour has invaded nearby structures, or has spread to lymph nodes or other organs.

Patients with prostate cancer may undergo watchful waiting of their cancer, or they may be treated by surgery, radiation therapy, high intensity focused ultrasound (HIFU), chemotherapy, cryosurgery, hormonal therapy, or some combination of these therapies. Patients with localised disease who are managed through watchful waiting have a high rate of progression-free survival^{13, 14} ; however, a significant number of males who choose watchful waiting will eventually progress to a more aggressive stage of prostate cancer, wherein treatment may be beneficial. Clinicians currently lack tools to accurately predict disease outcome and, accordingly, many prostate cancer patients undergo unnecessary aggressive local treatment, with significant morbidity, without any survival benefit¹⁵. Management by active surveillance with selective delayed intervention based on early PSA changes has been proposed as a strategy to reduce over-treatment of patients with indolent disease. However, although both baseline PSA measurements and rate of PSA change are important prognostic factors, they perform poorly in distinguishing those who will develop a fatal prostate cancer from those at low or no risk of disease progression¹⁶.

The present applicant has investigated whether MIC-1 represents a biomarker that could distinguish between patients with aggressive tumours from those with tumours that follow a benign course. To assess the predictive value of MIC-1 for prostate cancer progression, MIC-1 serum concentrations were measured in a large population-based cohort of incident prostate cancer patients with varying disease stage. It was surprisingly found that serum or plasma concentration of MIC-1 may be diagnostically and/or prognostically informative of prostate cancer, and as such, MIC-1 offers considerable potential as a valuable biomarker for predicting prostate cancer progression, and further, that elevated MIC-1 concentrations may be useful for determining appropriate treatment methods for prostate cancer. Additionally, the present applicant compared MIC-1 serum concentrations during prostate cancer with MIC-1 serum concentrations in healthy control population and, surprisingly, determined that in addition to being associated with age, elevated serum concentrations of MIC-1 were inversely associated with overall survival in apparently healthy subjects.

Accordingly, the present applicant has found that MIC-1 serum concentrations may be a useful tool for predicting mortality in prostate cancer patients as well as in the apparently healthy populations.

### SUMMARY OF THE INVENTION

In a first aspect, the present invention provides a method of prognosis of overall survival of an apparently healthy subject, the method comprising detecting an elevated amount of MIC-1 in a test body sample from said subject, wherein the elevated amount of MIC-1 is associated with an increased likelihood of death of the subject.

A second aspect, of the description provides a method of prognosis of prostate cancer in a male subject, the method comprising detecting an elevated amount of MIC-1 in a test body sample from the subject, wherein the elevated amount of MIC-1 is associated with an increased likelihood of prostate cancer progression.

A third aspect of the description provides a method of selecting subjects, who have been diagnosed with prostate cancer, who would benefit from active treatment for prostate cancer, the method comprising detecting an elevated amount of MIC-1 in a test body sample from the subject, wherein the elevated amount of MIC-1 indicates that the subject would benefit from active treatment for prostate cancer.

A fourth aspect of the description provides a method of selecting subjects for post-prostate cancer treatment adjuvant therapy, the method comprising detecting an elevated amount of MIC-1 in a test body sample from the subject, wherein the elevated amount of MIC-1 indicates that the subject would benefit from adjuvant therapy.

### BRIEF DESCRIPTION OF THE FIGURES

**Figure 1** provides a graph showing boxplots of MIC-1 serum concentrations (pg/mL) among unaffected control population by age;
**Figure 2** provides a graph showing MIC-1 serum concentrations by clinical stage of disease in prostate cancer cases;

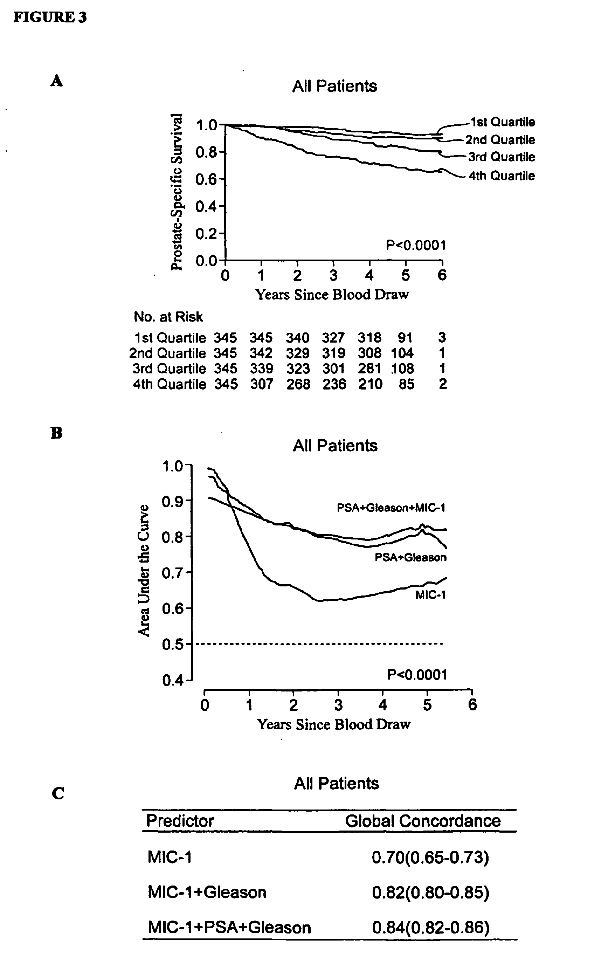

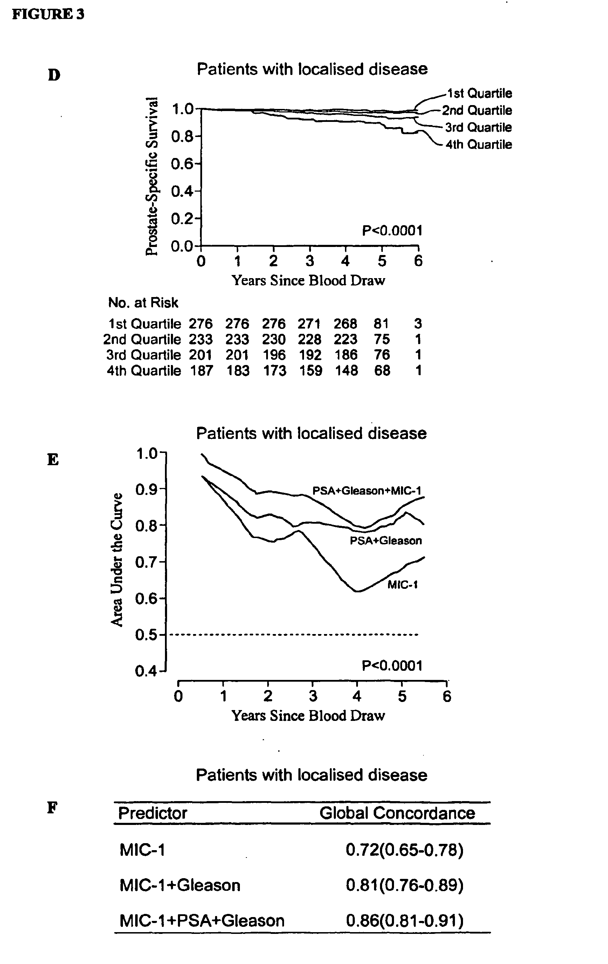

**Figure 3** provides graphs demonstrating the relationship between MIC-1 serum concentration and prostate-cancer-specific survival for either (A, B and C) all subjects or (D, E and F) subjects with localised disease. (A, D) Kaplan-Meier estimates of survival stratified by quartiles of MIC-1 serum concentrations. (B, E) Incident/dynamic area under curve plots demonstrating accuracy of MIC-1 serum concentration, combination of PSA and Gleason sum, and combination of MIC-1 serum concentration, PSA and Gleason sum as tests for fatal prostate cancer up to six years after blood draw, the lines plot estimates of area under curve versus time since blood draw based on a varying-coefficient multiplicative hazard model. (C, F) Global concordance summary for the predictive model including only MIC-1, the combination of PSA and Gleason sum, and the combination of MIC-1, PSA and Gleason sum. Nonparametric bootstrap based on re-sampling of covariates and survival observations was applied to determine confidence interval (CI) for the global concordance summary;
**Figure 4** provides Kaplan Meier plots demonstrating that serum MIC-1 level stratifies apparently healthy subjects from the all male control population cohort that died within the follow up period (A) when subjects are stratified by the serum MIC-1 median (82% with MIC-1 levels above the median survived compared to 94% of those with MIC-1 levels below the median; p<0.0001); and (B) when subjects are stratified by serum MIC-1 quartiles;
**Figure 5** provides Kaplan Meier plots demonstrating that serum MIC-1 level quartiles predict the risk of future mortality in the twin cohort;
**Figure 6** provides graphs demonstrating that serum MIC-1 level is significantly related to survival time and is independent of genetic background in (A) monozygotic twin pairs (MZ; i=0.419; p<0.0001) and (B) in dizygotic twin pairs (DZ; r=0.342; p=0.0046), and these correlations were not significantly different (ratio of relative risk=1.27; 95%CI=0.63-2.53); and
**Figure 7** provides a graph demonstrating the cumulative incidence of prostate cancer mortality stratified by quartiles of MIC-1 serum concentration among 1,442 prostate cancer patients.

### DETAILED DESCRIPTION OF THE INVENTION

The present applicant has surprisingly identified that serum MIC-1 is a powerful predictor of all cause mortality in apparently healthy subjects, which may identify patients at increased risk of mortality, potentially allowing investigation and intervention to improve quality of life and reduce health care costs.

Accordingly, in a first aspect, the present invention provides a method of prognosis of overall survival of an apparently healthy subject, the method comprising detecting an elevated amount of MIC-1 in a test body sample from said subject, wherein the elevated amount of MIC-1 is associated with an increased likelihood of death of the subject.

As used herein, the term "overall survival" is to be understood as referring to the survival of an apparently healthy subject; more particularly, that the subject does not die from any cause other than accident or misadventure (eg the subject does not die from a medical cause such as a life-threatening disease or condition such as cancer, particularly an epithelial cancer such as prostate cancer, and cardiovascular disease and events) or, in other words, the subject does not die from all cause mortality. The term "apparently healthy subject" as used herein, is to be understood as referring to a subject with no apparent symptoms or ill effects of life-threatening diseases or conditions (such as those mentioned above). Preferably, the subject is apparently healthy at the time of taking the test body sample from said subject.

In accordance with the first aspect of the present invention, it is to be understood that the elevated amount of MIC-1 in a test body sample predicts an increased likelihood of death from any cause other than accident or misadventure (ie the elevated amount of MIC-1 provides a prognosis of the likely death of the aparrently healthy subject). It is also to be understood that where there is no elevated amount of MIC-1 in the test body sample (eg where the amount of MIC-1 detected is in the range, or below, that which is considered to be normal), the method of the first aspect predicts that the subject has an increased likelihood of overall survival.

In some embodiments, the elevated amount of MIC-1 in a test body sample predicts an increased likelihood of death from cancer or a cardiovascular disease or other life-threatening medical events.

In some embodiments, the elevated amount of MIC-1 predicts an increased likelihood of death of the subject within a period of 10 years, or otherwise within 5 years, of the taking of the test body sample. In some embodiments, the elevated amount of MIC-1 predicts an increased likelihood of death of the subject within 3 years, or otherwise within 1 year, of the taking of the test body sample.

The amount of what may be regarded as an "elevated amount" of MIC-1 for the purposes of the method of the first aspect of the present invention, may vary according to the particular body sample type used and the age of the subject.

The preferred test body sample for use in the method of the first aspect is a sample of serum; however, a sample of amniotic fluid, placenta extract, whole blood, blood plasma, buffy coat, urine, cerebrospinal fluid, seminal fluid, synovial fluid, or a tissue biopsy may also be suitable. Persons skilled in the art will understand that an amount of MIC-1 in a body sample may be determined as a concentration or level of MIC-1 in said body sample. Further, persons skilled in the art will understand that the concentration of MIC-1 in a serum sample is substantially equivalent to the concentration of MIC-1 in a plasma sample since the major component of plasma is serum, with the difference merely constituting fibrinogen and other clotting factors. Moreover, persons skilled in the art will understand that the concentration of MIC-1 in a serum or plasma sample corresponds to approximately twice the concentration of MIC-1 in a whole blood sample, since whole blood comprises approximately half serum or plasma.

Accordingly, for a serum sample, an amount of >1 ng/mL is likely to represent an elevated amount of MIC-1 predicting that the subject has an increased likelihood of death from any cause other than accident or misadventure, while an amount of MIC-1 of >1.3 ng/mL is likely to represent an elevated amount of MIC-1 strongly predicting that the subject has an increased likelihood of death from any cause other than accident or misadventure. Further, an amount of MIC-1 of > 1.6 ng/mL in a serum sample is likely to represent an elevated amount of MIC-1 even more strongly predicting that the subject has an increased likelihood of death from any cause other than accident or misadventure.

The normal range of serum MIC-1 levels has previously been shown to be approximately from 0.2 to 1.150 ng/ml²⁹ ; however, the present applicant has shown that MIC-1 tends to increase with age.

Accordingly, in some embodiments, the amount of MIC-1 in a serum sample likely to represent an elevated amount of MIC-1 predicting that the subject has an increased likelihood of death from any cause other than accident or misadventure, is an amount in the top haptile of MIC-1 levels determined for age-matched apparently healthy subjects. As such, an amount of MIC-1 in a serum sample that is likely to represent an elevated amount of MIC-1 predicting that the subject has an increased likelihood of death from any cause other than accident or misadventure may be >0.543 ng/ml for 45 to 54 year olds, >0.626 ng/ml for 55 to 59 year olds, >0.831 ng/ml for 60 to 64 year olds, >0.926 ng/ml for 65 to 69 year olds, >1.025 ng/ml for 70 to 74 year olds, and >1.260 ng/ml for 75 to 79 year olds.

However, in preferred embodiments, the amount of MIC-1 in a serum sample likely to represent an elevated amount of MIC-1 predicting that the subject has an increased likelihood of death from any cause other than accident or misadventure, is an amount in the top quartile of MIC-1 levels determined for age-matched apparently healthy subjects. As such, an amount of MIC-1 in a serum sample that is likely to represent an elevated amount of MIC-1 predicting that the subject has an increased likelihood of death from any cause other than accident or misadventure may be >0.679 ng/ml for 45 to 54 year olds, >0.914 ng/ml for 55 to 59 year olds, >1.087 ng/ml for 60 to 64 year olds, >1.199 ng/ml for 65 to 69 year olds, >1.430 ng/ml for 70 to 74 year olds, and > 1.765 ng/ml for 75 to 79 year olds.

The amount of MIC-1 present in a test body sample may be readily determined by, for example, immunoassays such as enzyme-linked immunosorbant assay (ELISA) or immunohistochemistry (eg with sectionalised samples of a tissue biopsy) using anti-MIC-1 antibodies or fragments thereof. Anti-MIC-1 antibodies and fragments thereof may be produced by any of the methods well known to persons skilled in the art.

In an embodiment of the first aspect of the present invention, the elevated amount of MIC-1 in the test body sample is detected by:
(i) determining the amount of MIC-1 present in the said test body sample; and
(ii) comparing said amount of MIC-1 against an amount or a range of amounts of MIC-1 present in comparative body sample(s) taken from normal subject(s).

As used herein, the term "normal subject" refers to a subject who does not die from any cause other than accident or misadventure within 10 years of the taking of the comparative body sample(s).

In some embodiments, the normal subject(s) are age-matched, wherein the normal subject(s) are within 10 years of the age of the subject from which the relevant test body sample has been taken. More preferably, the normal subject(s) are within 5 years of the age of the subject from which the relevant test body sample has been taken.

It is to be understood that where an elevated amount of MIC-1 is detected in the test body sample, the greater the difference of that amount to that of the normal subject(s) the more strongly that elevated amount predicts that the subject has an increased likelihood of death from any cause other than accident or misadventure. Thus, in some embodiments, a difference in the amount of MIC-1 detected in the test body serum sample and that of the normal subject(s) of >0.3 ng/mL is likely to represent an elevated amount of MIC-1 indicating that the subject has an increased likelihood of death from any cause other than accident or misadventure, while a difference of >0.6 ng/mL is likely to represent an elevated amount of MIC-1 that more strongly indicates that the subject has an increased likelihood of death from any cause other than accident or misadventure.

In some embodiments of the first aspect of the present invention, the elevated amount of MIC-1 in a test body sample is an increase in the amount of MIC-1 within a subject detected using serial measurements (nb where a decrease in the amount of MIC-1 is detected following serial measurements, the method predicts that the subject has an increased likelihood of overall survival). Accordingly, the amount of MIC-1 in a test body sample may be determined at different time points in the same subject. For example, the amount of MIC-1 in a test body sample may be detected at certain time intervals. The time intervals may be determined on a case by case basis according to the needs of the subject. The time intervals may be, for example, three months, one year, five years or ten years, but it is to be understood that the time intervals may be adjusted according to any relevant health and medical factors of the subject. An elevated amount of MIC-1 in a test body sample within a subject can accordingly be detected by comparing the amount of MIC-1 in a test body sample at a given time point with the amount of MIC-1 in the same test body sample at an earlier time point. In this manner, an elevated amount of MIC-1 can be detected by determining the increase in the amount of MIC-1 present in the test body sample within any given subject over time.

Accordingly, in an embodiment of the first aspect of the present invention, the elevated amount of MIC-1 in the test body sample is an increase in the amount of MIC-1 within a subject detected using serial measurement by:
(i) determining the amount of MIC-1 present in the said test body sample; and
(ii) comparing said amount of MIC-1 against an amount or a range of amounts of MIC-1 present in comparative body sample(s) taken from the same subject at an earlier time point.

In such an embodiment, the increased amount of MIC-1 within a subject may be adjusted to compensate for the increase in MIC-1 normally associated with the increase in age of the subject.

It is to be understood that a larger increase in the amount of MIC-1 detected in the subject following serial measurements more strongly predicts that the subject has an increased likelihood of death from any cause other than accident or misadventure than a smaller increase. In some embodiments, an increase in the amount of MIC-1 within a subject detected using serial measurement of >0.3 ng/mL in a serum sample is likely to represent an elevated amount of MIC-1 indicating that the subject has an increased likelihood of death from any cause other than accident or misadventure, while an increase in the amount of MIC-1 within a subject detected using serial measurement of >0.6 ng/mL is likely to represent an elevated amount of MIC-1 that more strongly indicates that the subject has an increased likelihood of death from any cause other than accident or misadventure.

In some embodiments of the first aspect of the invention, the subject is male. In other embodiments of the first aspect of the invention, the subject is female.

Further, in some embodiments, the subject is more than 35 years of age or, more preferably, more than 45 years of age. However, in other embodiments, the subject may be more than 55 years of age, or more than 65 years of age, or even more than 75 years of age.

In a second aspect, also herein is described a method of prognosis of prostate cancer in a male subject, the method comprising detecting an elevated amount of MIC-1 in a test body sample from the subject, wherein the elevated amount of MIC-1 is associated with an increased likelihood of prostate cancer progression.

In some embodiments herein described, the elevated amount of MIC-1 is associated with an increased likelihood of progression to aggressive prostate cancer. As used herein, the term "aggressive prostate cancer" is to be understood as referring to prostate cancer that is likely to advance to a more life-threatening prostate cancer, that is, advance to a more severe and deleterious stage and/or metastasise. In some aggressive cancers, this may happen at a greater rate than generally occurs for less aggressive cancers, for example, aggressive cancers may advance to more severe and deleterious stages over the course of one or more years. In other examples of aggressive cancers, this may occur even more rapidly, such as over the course of one to three months.

In accordance with the second aspect herein described, the elevated amount of MIC-1 is associated with an increased likelihood of prostate cancer progression and, consequently, an increased likelihood of death of the subject due to prostate cancer.

In some embodiments herein described, the elevated amount of MIC-1 predicts a likelihood of death of the subject from the prostate cancer within a period of 10 years, or otherwise within 5 years, of the taking of the sample. In some embodiments, the elevated amount of MIC-1 predicts an increased likelihood of death of the subject within 3 years, or otherwise within 1 year of the taking of the test body sample.

The amount of what may be regarded as an "elevated amount" of MIC-1 for the purposes of the method of the second aspect herein described, may vary according to the particular body sample type used and the age of the subject.

The preferred test body sample for use in the method of the second aspect is a sample of serum; however, other body samples such as those mentioned above in relation to the method of the first aspect may also be suitable.

For a serum sample, an amount of >1 ng/mL is likely to represent an elevated amount of MIC-1 predicting prostate cancer progression and, consequently, an increased likelihood of death of the subject due to prostate cancer. Further, an amount of MIC-1 of >1.3 ng/mL in a serum sample is likely to represent an elevated amount of MIC-1 strongly predicting prostate cancer progression and an increased likelihood of death of the subject due to prostate cancer. Alternatively, an amount of >1.466 ng/mL in a serum sample is likely to represent an elevated amount of MIC-1 strongly predicting prostate cancer progression and an increased likelihood of death of the subject due to prostate cancer. Still further, an amount of MIC-1 of >1.6 ng/mL in a serum sample is likely to represent an elevated amount of MIC-1 even more strongly predicting prostate cancer progression and an increased likelihood of death of the subject due to prostate cancer.

In an embodiment of the second aspect of the description, the elevated amount of MIC-1 in the test body sample is detected by:
(i) determining the amount of MIC-1 present in the said test body sample; and
(ii) comparing said amount of MIC-1 against an amount or a range of amounts of MIC-1 present in comparative body sample(s) taken from normal subject(s).

In some embodiments, the age of the normal subject(s) is within 10 years of the age of the subject from which the relevant test body sample has been taken. More preferably, the normal subject(s) is within 5 years of the age of the subject from which the relevant test body sample has been taken.

It is to be understood that where an elevated amount of MIC-1 is detected in the test body sample, the greater the difference of that amount to that of the normal subject(s), the more strongly that elevated amount predicts that the prostate cancer subject has an increased likelihood of death from prostate cancer. Thus, in some embodiments, a difference in the amount of MIC-1 detected in the test body serum sample and that of the normal subject(s) of >0.3 ng/mL is likely to represent an elevated amount of MIC-1 indicating that the prostate cancer subject has an increased likelihood of death from prostate cancer, while a difference of >0.6 ng/mL is likely to represent an elevated amount of MIC-1 that more strongly indicates that the prostate cancer subject has an increased likelihood of death from prostate cancer.

In some embodiments of the second aspect of the description, the elevated amount of MIC-1 in a test body sample is an increase in the amount of MIC-1 within a subject detected using serial measurement. Accordingly, the amount of MIC-1 in a test body sample may be determined at different time points in the same subject. For example, the amount of MIC-1 in a test body sample may be detected in a subject prior to diagnosis with prostate cancer, or immediately following diagnosis with prostate cancer, and then at certain time intervals following diagnosis. The time intervals may be determined on a case by case basis according to the needs of the subject. The time intervals may be, for example, three months or one year or two years, but it is to be understood that the time intervals may be adjusted according to the disease stage, or other relevant health and medical factors, of the subject. An elevated amount of MIC-1 within a subject detected using serial measurement in a test body sample within a subject can accordingly be detected by comparing the amount of MIC-1 in the test body sample at a given time point with the amount of MIC-1 in the same test body sample at an earlier time point. In this manner, the elevated amount of MIC-1 can be detected by determining the increase in the amount of MIC-1 present in the test body sample within any given subject over time. Accordingly, in an embodiment of the second aspect of the description, the elevated amount of MIC-1 in the test body sample is an increase in the amount of MIC-1 within a subject detected using serial measurement by:
(i) determining the amount of MIC-1 present in the said test body sample; and
(ii) comparing said amount of MIC-1 against an amount or a range of amounts of MIC-1 present in comparative body sample(s) taken from the same subject at an earlier time point.

In such an embodiment, the increased amount of MIC-1 within a subject may be adjusted to compensate for the increase in MIC-1 normally associated with the increase in age of the subject.

It is to be understood that a larger increase in the amount of MIC-1 detected in the subject following serial measurements more strongly predicts prostate cancer progression and, consequently, an increased likelihood of death of the subject due to prostate cancer, than a smaller increase. In some embodiments, an increase in the amount of MIC-1 within a subject detected using serial measurement of >0.3 ng/mL in a serum sample is likely to represent an elevated amount of MIC-1 strongly predicting prostate cancer progression and an increased likelihood of death of the subject due to prostate cancer, while an increase in the amount of MIC-1 within a subject detected using serial measurement of >0.6 ng/mL is likely to represent an elevated amount of MIC-1 even more strongly predicting prostate cancer progression and an increased likelihood of death of the subject due to prostate cancer.

In some embodiments of the second aspect of the description, the subject is more than 35 years of age or, more preferably, more than 45 years of age. However, in some embodiments the subject may be more than 55 years of age, or more than 65 years of age, or even more than 75 years of age.

The results of the method of the second aspect of the description may be used in combination with one or more other prognostic indicators (eg Gleason sum, PSA, TMN stage). In addition, using the results of the method in combination with an evaluation of MIC-1 stromal staining of prostate cancer tissues cores (as described in Bauskin et al. (2005) Cancer Res 65(6) 2330- 2336³⁰), may allow additional prognosis capacity between fatal and non-fatal localised prostate cancer (ie organ-confined prostate cancer). MIC-1 stromal staining of prostate cancer tissues may be performed using any of the suitable methods well known to persons skilled in the art including, for example, by immunohistochemistry using an anti-MIC-1 antibody. Accordingly, the method may further comprise detecting the elevated amount of MIC-1 in combination with one or more prognostic factors selected from the group consisting of Gleason sum, prostate specific antigen amount, stromal staining for MIC-1 and tumour-node-metastasis stage.

Subjects with prostate cancer may undergo watchful waiting of their cancer, or they may be treated by any method including surgery, radiation therapy, high intensity focused ultrasound (HIFU), chemotherapy, cryosurgery, hormonal therapy, gene therapy, vaccination, cytokine or cytokine modulation therapy (eg antibody therapy), or some combination of these therapies.

Accordingly, in a third aspect, the present description provides a method of selecting subjects, who have been diagnosed with prostate cancer, who would benefit from active treatment for prostate cancer, the method comprising detecting an elevated amount of MIC-1 in a test body sample from the subject, wherein the elevated amount of MIC-1 indicates that the subject would benefit from active treatment for prostate cancer.

In some embodiments herein described, the elevated amount of MIC-1 indicates that the subject would benefit from active treatment for prostate cancer. In other embodiments, the elevated amount of MIC-1 strongly indicates that the subject would benefit from active treatment for prostate cancer.

As used herein, the term "active treatment for prostate cancer" is to be understood as referring to treatment for prostate cancer that may remove and/or control the disease, such as the removal of the entire prostate gland. Such active treatment may be associated with undesirable side effects but may prevent the prostate cancer from advancing to a more life-threatening prostate cancer, that is advance to a more severe and deleterious stage and/or metastasise. Active treatments may include surgery, radiation therapy, high intensity focused ultrasound (HIFU), chemotherapy, cryosurgery, hormonal therapy, gene therapy, vaccination, cytokine or cytokine-modulation therapy (eg antibody therapy), or some combination of these therapies.

In some embodiments herein described, the subject is newly diagnosed with prostate cancer.

The preferred test body sample for use in the method of the third aspect is a sample of serum; however, other body samples such as those mentioned above in relation to the method of the first aspect may also be suitable.

The amount of what may be regarded as an "elevated amount" of MIC-1 for the purposes of the method of the third aspect of the present description, may vary according to the particular body sample type used and the age of the subject as described above for the first aspect of the invention and second aspect of the description.

For a serum sample from a subject diagnosed with prostate cancer, an amount of MIC-1 of >1 ng/mL is likely to represent an elevated amount of MIC-1 indicating that the subject would benefit from active treatment for prostate cancer. Further, an amount of MIC-1 of >1.3 ng/mL in a serum sample is likely to represent an elevated amount of MIC-1 strongly indicating that the subject would benefit from active treatment for prostate cancer; while an amount of MIC-1 of >1.6 ng/mL is likely to represent an elevated amount of MIC-1 even more strongly indicating that the subject would benefit from active treatment for prostate cancer.

The amount of MIC-1 present in a test body sample may be readily determined as described for the first aspect of the invention and the second aspect of the description.

In an embodiment of the third aspect of the present description, the elevated amount of MIC-1 in the test body sample is detected by:
(i) determining the amount of MIC-1 present in the said test body sample; and
(ii) comparing said amount of MIC-1 against an amount or a range of amounts of MIC-1 present in comparative body sample(s) taken from normal subject(s).

In some embodiments herein described, the age of the normal subject(s) is within 10 years of the age of the subject from which the relevant test body sample has been taken. More preferably, the normal subject(s) is within 5 years of the age of the subject from which the relevant test body sample has been taken.

In some embodiments of the third aspect of the present description, the elevated amount of MIC-1 in a test body sample is an increase in the amount of MIC-1 within a subject detected using serial measurement. Accordingly, the amount of MIC-1 in a test body sample may be determined at different time points in the same subject. For example, the amount of MIC-1 in a test body sample may be detected in a subject prior to diagnosis with prostate cancer, or immediately following diagnosis with prostate cancer, and then at certain time intervals following diagnosis. The time intervals may be determined on a case by case basis according to the needs of the subject. The time intervals may be, for example, three months or one year or two years, but it is to be understood that the time period may be adjusted according to the disease stage, or any other relevant health and medical factors, of the subject. An elevated amount of MIC-1 in a test body sample within a subject can accordingly be detected by comparing the amount of MIC-1 in a test body sample at a given time point with the amount of MIC-1 in the same test body sample at an earlier time point. In this manner, the elevated amount of MIC-1 can be detected by determining the increase in the amount of MIC-1 present within any given subject over time.

Accordingly, in an embodiment of the third aspect of the present description, the elevated amount of MIC-1 in the test body sample is an increase in the amount of MIC-1 within a subject detected using serial measurement by:
(i) determining the amount of MIC-1 present in the said test body sample; and
(ii) comparing said amount of MIC-1 against an amount or a range of amounts of MIC-1 present in comparative body sample(s) taken from the same subject at an earlier time point.

In such an embodiment, the increased amount of MIC-1 within a subject may be adjusted to compensate for the increase in MIC-1 normally associated with the increase in age of the subject.

It is to be understood that a larger increase in the amount of MIC-1 detected in the subject following serial measurements more strongly indicates that the subject may benefit from active treatment for prostate cancer. In some embodiments, an increase in the amount of MIC-1 within a subject detected using serial measurement of >0.3 ng/mL in a serum sample is likely to represent an elevated amount of MIC-1 strongly indicating that the subject may benefit from active treatment for prostate cancer, while an increase in the amount of MIC-1 within a subject detected using serial measurement of >0.6 ng/mL is likely to represent an elevated amount of MIC-1 strongly indicates that the subject would benefit from active treatment for prostate cancer.

In some embodiments of the third aspect of the present description, the subject is more than 35 years of age or, more preferably, more than 45 years of age. However, in some embodiments the subject may be more than 55 years of age, or more than 65 years of age, or even more than 75 years of age.

The results of the method of the third aspect of the descriptionmay be used in combination with one or more other prognostic indicators (eg Gleason sum and PSA). In addition, using the results of the method in combination with an evaluation of MIC-1 stromal staining may allow additional capacity to select a treatment strategy. Accordingly, the method may further comprise detecting the elevated amount of MIC-1 in combination with one or more prognostic factors selected from the group consisting of Gleason sum, prostate specific antigen amount, MIC-1 stromal staining and tumour-node-metastasis stage.

The present applicant has also observed that MIC-1 serum levels may remain elevated in prostate cancer patients even following active treatment (ie the elevated MIC-1 levels may be due to residual, undetected cancer) such as surgery or radiation therapy. In such cases, measurement of such posttreatment elevated MIC-1 levels may indicate those subjects that may benefit from adjuvant therapy.

Thus, in a fourth aspect, the present description provides a method of selecting subjects for post-prostate cancer treatment adjuvant therapy, the method comprising detecting an elevated amount of MIC-1 in a test body sample from the subject, wherein the elevated amount of MIC-1 indicates that the subject would benefit from adjuvant therapy.

As used herein, the term "adjuvant therapy" is to be understood as referring to an additional treatment for prostate cancer that may remove and/or control the disease, such as the removal of the entire prostate gland. Such adjuvant therapy may be associated with undesirable side effects but may prevent the prostate cancer from advancing to a more life-threatening prostate cancer, that is advance to a more severe and deleterious stage and/or metastasise. Adjuvant therapy may include surgery, radiation therapy, high intensity focused ultrasound (HIFU), chemotherapy, cryosurgery, hormonal therapy, gene therapy, vaccination, cytokine or cytokine-modulation therapy (eg antibody therapy), or some combination of these therapies.

The preferred test body sample for use in the method of the fourth aspect is a sample of serum; however, other body samples such as those mentioned above in relation to the method of the first aspect may also be suitable.

The amount of what may be regarded as an "elevated amount" of MIC-1 for the purposes of the method of the fourth aspect of the present description, may vary according to the particular body sample type used and the age of the subject as described above for the first aspect of the invention and the second aspect of the description.

For a serum sample from a subject diagnosed with prostate cancer, an amount of MIC-1 of >1 ng/mL is likely to represent an elevated amount of MIC-1 indicating that the subject would benefit from adjuvant therapy for prostate cancer. Further, an amount of MIC-1 of >1.3 ng/mL in a serum sample is likely to represent an elevated amount of MIC-1 strongly indicating that the subject would benefit from adjuvant therapy for prostate cancer; while an amount of MIC-1 of >1.6 ng/mL is likely to represent an elevated amount of MIC-1 even more strongly indicating that the subject would benefit from adjuvant therapy for prostate cancer.

The amount of MIC-1 present in a test body sample may be readily determined as described for the first aspect of the invention and the second aspect of the description.

In an embodiment of the fourth aspect of the present description, the elevated amount of MIC-1 in the test body sample is detected by:
(i) determining the amount of MIC-1 present in the said test body sample; and
(ii) comparing said amount of MIC-1 against an amount or a range of amounts of MIC-1 present in comparative body sample(s) taken from normal subject(s).

In some embodiments described herein, the age of the normal subject(s) is within 10 years of the age of the subject from which the relevant test body sample has been taken. More preferably, the normal subject(s) is within 5 years of the age of the subject from which the relevant test body sample has been taken.

In some embodiments of the fourth aspect of the present description, the elevated amount of MIC-1 in a test body sample is an increase in the amount of MIC-1 within a subject detected using serial measurement. Accordingly, the amount of MIC-1 in a test body sample may be determined at different time points in the same subject. For example, the amount of MIC-1 in a test body sample may be detected in a subject prior to diagnosis with prostate cancer, or immediately following diagnosis with prostate cancer, and then at certain time intervals following diagnosis, as well as at certain times following treatment for prostate cancer. The time intervals may be determined on a case by case basis according to the needs of the subject. The time intervals may be, for example, three months or one year or two years, but it is to be understood that the time period may be adjusted according to the disease stage, or any other relevant health and medical factors and treatment, of the subject. An elevated amount of MIC-1 in a test body sample within a subject can accordingly be detected by comparing the amount of MIC-1 in a test body sample at a given time point with the amount of MIC-1 in the same test body sample at an earlier time point. In this manner, the elevated amount of MIC-1 can be detected by determining the increase in the amount of MIC-1 present within any given subject over time.

Accordingly, in an embodiment of the fourth aspect of the present description, the elevated amount of MIC-1 in the test body sample is an increase in the amount of MIC-1 within a subject detected using serial measurement by:
(i) determining the amount of MIC-1 present in the said test body sample; and
(ii) comparing said amount of MIC-1 against an amount or a range of amounts of MIC-1 present in comparative body sample(s) taken from the same subject at an earlier time point.

In such an embodiment, the increased amount of MIC-1 within a subject may be adjusted to compensate for the increase in MIC-1 normally associated with the increase in age of the subject.

It is to be understood that a larger increase in the amount of MIC-1 detected in the subject following serial measurements more strongly indicates that the subject may benefit from adjuvant therapy for prostate cancer. In some embodiments, an increase in the amount of MIC-1 within a subject detected using serial measurement of >0.3 ng/mL in a serum sample is likely to represent an elevated amount of MIC-1 strongly indicating that the subject may benefit from adjuvant therapy for prostate cancer, while an increase in the amount of MIC-1 within a subject detected using serial measurement of >0.6 ng/mL is likely to represent an elevated amount of MIC-1 strongly indicates that the subject would benefit from adjuvant therapy for prostate cancer.

In some embodiments of the fourth aspect of the present description, the subject is more than 35 years of age or, more preferably, more than 45 years of age. However, in some embodiments the subject may be more than 55 years of age, or more than 65 years of age, or even more than 75 years of age.

The results of the method of the fourth aspect of the descriptionmay be used in combination with one or more other prognostic indicators (eg Gleason sum and PSA). In addition, using the results of the method in combination with an evaluation of MIC-1 stromal staining may allow additional capacity to select a treatment strategy. For example, it has previously been shown that MIC-1 stromal staining levels are linked to prostate cancer outcome following radical prostatectomy, with decreasing stromal levels providing an important independent predictor of disease relapse³⁰. Accordingly, the method may further comprise detecting the elevated amount of MIC-1 in combination with one or more prognostic factors selected from the group consisting of Gleason sum, prostate specific antigen amount, MIC-1 stromal staining and tumour-node-metastasis stage.

The invention will hereinafter be described by way of the following non-limiting example and accompanying figures.

### EXAMPLES

### Example 1 Serum concentrations of MIC-1 in healthy control population and prostate cancer patients - for illustration only

### Materials and Methods

### Prostate Cancer Study Population

The prostate cancer population was part of a population-based case-control study of prostate cancer aetiology known as the Cancer Prostate in Sweden (CAPS) study, which was conducted in two phases with enrolment between January 2001 and October 2003. Briefly, subjects were all men between 35 and 79 years of age with pathologically verified adenocarcinoma of the prostate (ICD-10: C61). Serum samples from 1380 prostate cancer cases were retrieved for MIC-1 serum analysis. Clinical information such as tumour-node-metastasis (TNM) stage, Gleason sum, diagnostic prostate-specific antigen (PSA) concentration, and primary treatment was obtained through linkage to the National Prostate Cancer Registry (Table 1). Prostate cancer patients donated blood, on average 4.9 months (range 0.7 to 23.7 months) after the date of diagnosis, which were stored at -70°C until analysis.

### Apparently Healthy Control Population

876 male, unaffected, apparently healthy, control population subjects were randomly selected from the Swedish Population Registry, and frequency matched to the expected distribution of the prostate cases described above by age (in 5-year age categories) and geographic residence. Cases were all men between 35 and 79 years of age. Serum samples from the 876 control population subjects were retrieved for MIC-1 serum analysis.

**Table 1. Descriptive Characteristics of the Study Cohort.***

| Characteristic | Alive (n=1,064) | Deceased from other events (n=105) | Deceased from prostate cancer. (n=211) |
|---|---|---|---|
| Age (years) | 66.2±7.1 | 71.6±6.3 | 68.9±7.4 |
| PSA levels, ng/ml | | | |
| <20 | 731 (69) | 60 (57) | 43 (20) |
| 20-49 | 168 (16) | 16 (15) | 43 (20) |
| ≥50 | 135 (13) | 27 (26) | 120 (57) |
| Missing | 30 (3) | 2 (2) | 5 (2) |
| Clinical stage^{†} | | | |
| I | 24 (2) | 3 (3) | 0 |
| II | 771 (72) | 58 (55) | 41 (19) |
| III | 177 (17) | 32 (30) | 53 (25) |
| IV | 71 (7) | 10 (10) | 114 (54) |
| Missing | 21 (2) | 2 (2) | 3 (1) |
| Gleason score | | | |
| 2-6 | 576 (54) | 52 (50) | 11 (5) |
| 7 | 287 (27) | 28 (27) | 64 (30) |
| 8-10 | 107 (10) | 19 (18) | 92 (44) |
| Missing | 94 (9) | 6 (6) | 44 (21) |
| Primary treatment | | | |
| Watchful waiting | 197 (19) | 28 (27) | 12 (6) |
| Curative | 599 (56) | 32 (30) | 29 (14) |
| Palliative | 268 (25) | 45 (43) | 170 (81) |
| MIC-1 serum concentration (pg/ml) | 1066±602 | 1745±1206 | 2265±3101 |

| | | | |
|---|---|---|---|
| * Plus-minus values are means ±SD. † Clinical stage grouped according to the International Union Against Cancer TNM classification of malignant tumors.¹² | | | |

### Follow-up assessment

Complete follow-up for prostate cancer specific mortality was achieved up until 1 March 2007 through record linkage to the Swedish Cause of Death Registry using each study participant's unique national registration number. Review of death certificates, performed by an experienced oncologist, established the cause of death for subjects deceased after 31 December 2004, with prostate cancer specific death defined as those who had prostate cancer classified as the underlying cause of death. The average follow-up time was 4.6 years (range 0.6 to 6.5 years). A total of 325 (23%) prostate cancer patients died during follow-up and of those 218 (15%) had prostate cancer classified as their underlying cause of death. Among the unaffected control population, 82 (9%) died during follow-up.

### Determination of MIC-1 serum levels

MIC-1 serum levels were determined using a MIC-1 sandwich ELISA. The sandwich ELISA was established using the mouse monoclonal antibody (MAb) 26G6H6^{2,3} for antigen capture and a sheep polyclonal antibody (PAb) 233B3-P for detection². The optimum concentration of both antibodies was determined and then used for all subsequent studies. Ninety-six-well Maxisorp ELISA plates were coated with MAb 26G6H6 supernatant diluted 1:5 (final concentration was approximately 20ng/mL) in coating buffer (0.1 mol/L carbonate in distilled water, pH 9.4-9.8) at 4°C for 24 hours. ELISA plates were then washed three times with 300 µL/well 1% (wt/vol) bovine serum albumin (BSA) in phosphate buffered saline (PBS) for 2 h at 37°C. Recombinant human MIC-1 (rhMIC-1) standards, tissue culture supernatant, or patient serum were then added to the plates (100µL/well) and incubated for 1 h at 37°C. The plates were washed three times, followed by addition of 100µL/well of the sheep PAb 233B3-P diluted 1:5000 in antibody diluent (Ab dil; PBS containing 1% (wt/vol) BSA and 0.05% (vol/vol) Tween-20) and incubated for 1 h at 37°C. ELISA plates were then washed three times, and 100µL/well of biotinylated donkey anti-sheep IgG diluted to 1:5000 Ab dil was added and incubated for 1 h at 37°C. The plates were washed four times, followed by the addition of 100µL/well of peroxidase substrate (1 mg/mL o-phenylenediamine dihydrochloride (Sigma)) in 0.05 mol/L phosphate-citrate buffer containing 0.014% H₂O₂, pH5.0 (Sigma). Colour development was allowed to proceed for 5-15 min and was terminated by the addition of 100µL/well of 4N H₂SO₄. The absorbance was measured at 490 nm in a microplate reader. The concentration of human MIC-1 (hMIC-1) in the samples was determined by comparison with the rhMIC-1 standard curve. The standard curve was constructed using standard curve-fitting software supplied with the microplate reader (Pasteur Diagnostics). The concentration of rhMIC-1 in the standard curve was determined on the basis of a comparison of this standard to a master standard of highly purified recombinant MIC-1. The master standard protein concentration was determined by an average of eight estimations of total amino acid composition. All samples were assayed in triplicate on at least two occasions. Results are presented as the mean +/- SD. The serum samples were labelled blindly for the determination of serum concentrations.

### Statistical Analysis

MIC-1 serum levels were compared by age using ANOVA analysis, with MIC-1 concentration levels log-transformed. Survival was assessed from date of diagnosis until date of death or until date of censoring (1 March 2007). Survival time was censored at time of death for patients dying from causes other than prostate cancer. Cox regression models were fitted to assess hazard ratios (HR) of prostate cancer mortality by MIC-1 serum levels.

### Results

### Association between MIC-1 serum concentration in unaffected control population and age

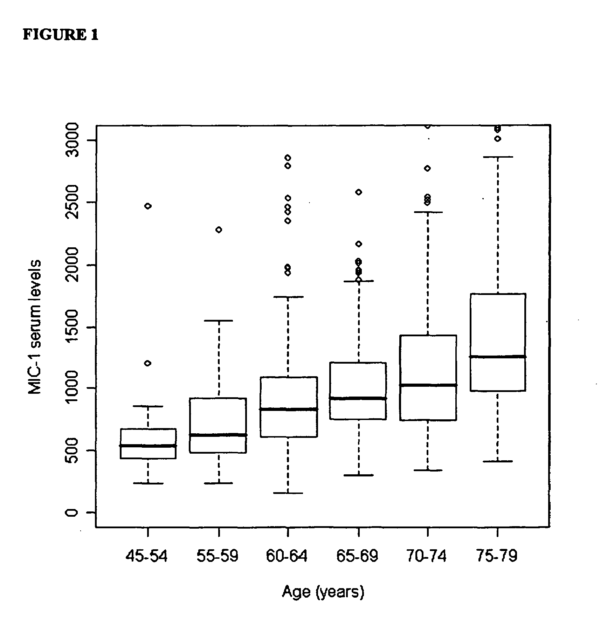

As shown in Table 2 and Figure 1, MIC-1 serum concentration strongly correlates with age in unaffected, apparently healthy control population subjects. For example, the mean ± SD MIC-1 serum concentration for 45-54 year olds was 543 ± 352 pg/ml; whereas it was 1260 ± 1033 pg/ml in the 75-79 year olds.

**Table 2 Descriptive statistics of MIC-1 serum concentrations among control population**

| **Age** | **No.** | **Min** | **25% qu** | **Mean** | **Median** | **75% qu** | **Max** | **Interquartile range** | **SD** |
|---|---|---|---|---|---|---|---|---|---|
| 45-54 | 40 | 239 | 439 | 613 | 543 | 679 | 2467 | 240 | 352 |
| 55-59 | 88 | 235 | 486 | 762 | 626 | 914 | 3983 | 428 | 496 |
| 60-64 | 192 | 156 | 614 | 967 | 831 | 1087 | 4052 | 472 | 604 |
| 65-69 | 174 | 301 | 761 | 1177 | 926 | 1199 | 9638 | 438 | 1136 |
| 70-74 | 194 | 341 | 750 | 1190 | 1025 | 1430 | 4710 | 680 | 687 |
| 75-79 | 188 | 414 | 976 | 1551 | 1260 | 1765 | 7825 | 789 | 1033 |
| | | | | | | | | | |
| Overall P | 5,6E-41 | | | | | | | | |

| | | | | | | | | | |
|---|---|---|---|---|---|---|---|---|---|
| ¹**ANOVA** | | | | | | | | | |

Association between MIC-1 serum concentration in unaffected control population and overall survival As shown in Table 3, MIC-1 serum concentration surprisingly showed a strong inverse correlation with overall survival in the control population cohort. For example, when the control population was stratified into quartiles according to serum MIC-1 level, only 3% of the control population with a MIC-1 serum concentration of <673 pg/ml died from any cause; whilst 22% of the control population with a MIC-1 serum concentration of >1299 pg/ml died from any cause.

**Table 3 Hazard ratios by MIC-1 serum concentrations for death from any causes among 876 control population Deaths**

| **Predictor MIC-1 (pg/mL)** | **No. Subjects** | **No.** | **(%)** | **HR¹** | **95% CI** | | **HR²** | **95% CI** | |
|---|---|---|---|---|---|---|---|---|---|
| <673 | 219 | 6 | 3% | 1,00 | | | 1,00 | | |
| 673-934 | 219 | 14 | 6% | 2,40 | 0,92 | 6,25 | 1,97 | 0,74 | 5,24 |
| 935-1299 | 219 | 14 | 6% | 2,38 | 0,91 | 6,19 | 1,57 | 0,58 | 4,27 |
| >1299 | 219 | 48 | 22% | 8,83 | 3,78 | 20,64 | 5,23 | 2,11 | 12,96 |

| | | | | | | | | | |
|---|---|---|---|---|---|---|---|---|---|
| ¹ Hazard ratios from Cox models ² Hazard ratios from Cox models adjusted for age | | | | | | | | | |

### Prostate cancer patient cohort and follow-up

In total, 414 (30%) of the prostate cancer cases were discovered through an elevated PSA concentration in a PSA test; and 897 (65%) of the patients were diagnosed with a localised disease, wherein the cancer was confined within the prostate capsule with no evidence of regional or distant spread (Table 1). The majority of the patients received initial treatment; 48% of the study cohort were primarily treated with curative intention and 35% with palliative intention. During follow-up, 316 (23%) of the 1380 men died and of those 218 (15%) had prostate cancer classified as their underlying cause of death. The average follow-up time was 4.7 years (range 0.1 to 5.9 years).

### MIC-1 serum concentrations and clinical stage of prostate cancer disease

MIC-1 serum concentrations differed significantly across different clinical stages of prostate cancer (P<0.001). Significantly elevated MIC-1 serum concentrations were observed among patients with locally advanced stage III prostate cancer (mean = 1394 pg/ml, p<0.001) and among patients with metastatic stage IV prostate cancer (mean = 2084 pg/ml, p<0.001) as compared to patients with localised stage I-II disease (mean = 1101 pg/ml).

### MIC-1 serum concentrations and prostate cancer death

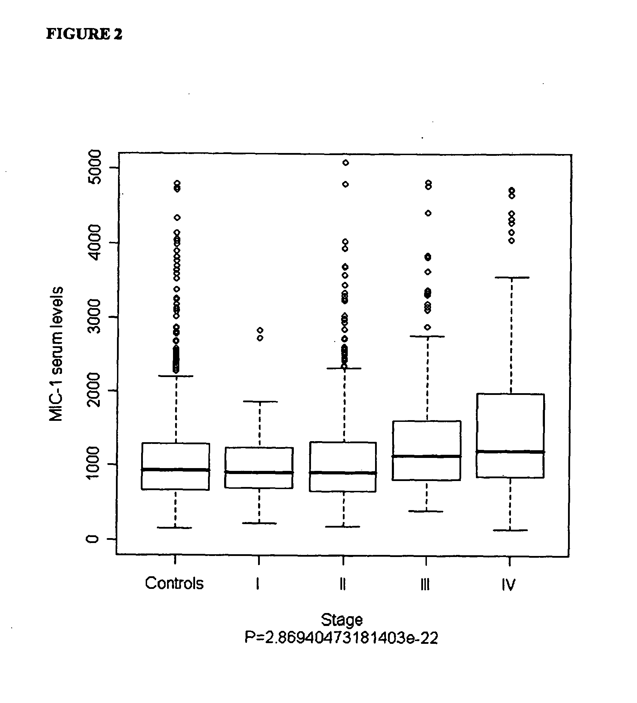

Prostate cancer patients were stratified into quartiles according to serum MIC-1 levels. The distribution of MIC-1 serum concentrations in patients who ultimately died of prostate cancer were skewed toward the highest quartile compared to surviving patients (Figure 2). Univariate Cox regression analysis revealed a strong association between increasing concentrations of MIC-1 and higher death rates, with each 100% increment in log-transformed MIC-1 concentration being associated with a four-fold higher death rate (P for trend <0.001). Whilst only 6% of patients with MIC-1 serum concentrations <722 pg/mL died during follow-up, 30% of patients with MIC-1 serum concentrations >1466 pg/mL died, yielding a 6-fold gradient (hazard ratio = 6.1, 95% confidence interval (CI) = 3.8 to 9.8; Table 4).

Adjustment for Gleason sum, TNM stage, and PSA concentration attenuated the strength of association between MIC-1 serum concentrations and prostate cancer survival. However, higher MIC-1 concentrations remained an independent predictor of prognosis with a more than three-fold higher death rate in the highest compared with the lowest category (hazard ratio = 3.4, 95%CI = 2.0 to 5.8; Table 4).

Compared with the total study cohort, an even stronger association was observed between MIC-1 serum concentrations and prostate cancer survival among patients with localised disease. Patients with the highest serum MIC-1 concentrations encountered an 11-fold higher death rate than those in the lowest category (hazard ratio = 11.4, 95%CI = 3.4 to 38.3). In adjusted analysis, MIC-1 remained an independent prognostic factor with an almost six-fold higher death rate in the highest compared with the lowest category (hazard ratio = 5.8, 95%CI = 1.7 to 20.2).

### Predictive accuracy of MIC-1 serum concentrations for prostate cancer outcome

MIC-1 serum concentrations showed good predictive accuracy in classifying fatal from nonfatal prostate cancer for early follow-up times; however, the discriminatory capacity gradually decreased with time to approximately 0.68 at end of follow-up resulting in a global concordance summary of 0.70 (95%CI = 0.65 to 0.72; Figure 3). The global concordance summary increased significantly from 0.82 for the predictive model including PSA and Gleason sum to 0.84 for the predictive model which also included MIC-1 (p < 0.001). Among patients diagnosed with localised disease, the global concordance summary increased significantly from 0.82 to 0.86 (p < 0.001) when MIC-1 was included in addition to PSA and Gleason sum in the predictive model.

**Table 4 Hazard ratios by MIC-1 serum levels for death from prostate cancer among 1380 prostate cancer patients.**

| | | **Frequency of Prostate Cancer Deaths** | | | | | |
|---|---|---|---|---|---|---|---|
| **MIC-1 serum concentration (pg/ml)** | **Number of patients** | ***No*. *of patients*** | ***proportion*** | ***HR¹*** | **95%CI** | **HR²** | **95%CI** |
| **MIC-1** | | | | | | | |
| <722 | 345 | 21 | 0.06 | 1.0 | | 1.0 | |
| 722-1015 | 345 | 32 | 0.09 | 1.6 | 0.9-2.7 | 1.1 | 0.6-1.9 |
| 1016-1466 | 345 | 56 | 0.16 | 2.8 | 1.7-4.7 | 1.8 | 1.0-3.0 |
| >1466 | 345 | 102 | 0.30 | 6.1 | 3.8-9.8 | 3.4 | 2.0-5.8 |
| P trend | | | | <0.001 | | <0.001 | |
| | | | | | | | |

| **Gleason sum** | | | | | | | |
|---|---|---|---|---|---|---|---|
| 2-6 | 673 | 18 | 0.03 | 1.0 | | 1.0 | |
| 7 | 444 | 87 | 0.20 | 8.1 | 4.9-13.4 | 4.0 | 2.3-6.8 |
| 8-10 | 233 | 101 | 0.43 | 22.4 | 13.5-37.0 | 7.9 | 4.5-13.8 |
| P trend | | | | <0.001 | | <0.001 | |
| | | | | | | | |

| **T stage** | | | | | | | |
|---|---|---|---|---|---|---|---|
| T1-T2 | 947 | 62 | 0.07 | 1.0 | | 1.0 | |
| T3-T4 | 406 | 146 | 0.36 | 6.8 | 5.1-9.2 | 1.9 | 1.4-2.7 |
| | | | | | | | |

| **PSA** | | | | | | | |
|---|---|---|---|---|---|---|---|
| 0-19 | 834 | 43 | 0.05 | 1.0 | | 1.0 | |
| 20-49 | 227 | 43 | 0.19 | 4.0 | 2.6-6.1 | 1.8 | 1.2-2.9 |
| 50+ | 282 | 120 | 0.43 | 11.0 | 7.8-15.6 | 2.0 | 1.3-3.0 |
| P trend | | | | <0.001 | | <0.001 | |
| | | | | | | | |

| **Metastatic** | | | | | | | |
|---|---|---|---|---|---|---|---|
| Organ | 1188 | 104 | 0.09 | 1.0 | 0 | 1.0 | |
| confined | | | | | | | |
| N+ | 39 | 15 | 0.38 | 5.1 | 2.9-8.7 | 3.2 | 1.8-5.7 |
| M+ | 134 | 90 | 0.67 | 13.2 | 9.9-17.6 | 4.2 | 3.0-5.9 |
| P trend | | | | <0.001 | | <0.001 | |

| | | | | | | | |
|---|---|---|---|---|---|---|---|
| ¹Hazard ratios from univariate Cox models ²Hazard ratios from a multiple Cox model including MIC-1, Gleason sum, TNM stage, PSA at diagnosis, and presence of regional or distant metastases as covariates. | | | | | | | |

### Discussion

The studies described in this example confirm the association between MIC-1 serum concentrations and disease stage and demonstrate the prognostic value of serum MIC-1 concentration in a large population-based cohort of prostate cancer patients. In multivariate analysis, adjusted for important prognostic factors including Gleason sum, clinical stage, and diagnostic PSA concentration, MIC-1 remained an independent prognostic indicator of disease outcome.

Importantly, in organ-confined disease, an elevated serum MIC-1 concentration was a strong, independent predictor of ultimately fatal prostate cancer. The predictive value of serum MIC-1 concentrations was further enhanced when traditional markers of disease (Gleason sum and PSA) were also used to classify fatal from non-fatal prostate cancer. The prognostic value of serum MIC-1, Gleason sum and PSA in initially localised prostate cancer was especially pronounced. These results strongly indicate that serum MIC-1 concentration is an important biomarker capable of predicting prostate cancer progression.

The studies also show that combining serum MIC-1 concentration with PSA concentration and Gleason sum significantly improves the accuracy of prognosing disease outcome, especially among patients with localised disease. Specifically, this improvement was most pronounced in early-event predictions with a gradual decrease in predictive benefit with increasing follow-up time. Therefore, a high diagnostic MIC-1 concentration may be used to identify patients that may benefit from primary systemic adjuvant treatment in addition to local treatment.

Despite the strong relationship of MIC-1 with cancer, its role in tumourigenesis is not well understood⁶. The majority of studies report an antitumourigenic role of MIC-1 both in regulating tumour growth^{9, 17, 18}, through induction of apoptosis via both p53-dependent and p53-independent pathways, and through antiangiogenic activity¹⁹; however, enhancement of tumourigenic activity has also been reported²⁰. In the present studies, a significant association was observed between MIC-1 serum concentrations and prostate cancer-specific survival. The consistent direction of the association between serum MIC-1 concentrations and prostate cancer death suggest a functional role of MIC-1 in prostate cancer progression.

In conclusion, serum concentrations of MIC-1 were markedly elevated in prostate cancer patients with locally advanced and metastatic disease. In addition, serum MIC-1 concentrations were a strong predictor of prostate cancer death, independent of known prognostic factors, particularly among patients with disease confined to the prostate gland. Further, serum MIC-1 concentrations showed a strong correlation with age and, moreover, overall survival among the control population.

### Example 2 Further examination of association of serum MIC-1 with survival in the apparently healthy male control population cohort

### Materials and Methods

### Follow-up assessment of male control population subjects

For the initial cohort of 876 apparently healthy men described above, complete follow-up for specific mortality was achieved up until 1 March 2007 through record linkage to the Swedish Cause of Death Registry using each study participant's unique national registration number. Review of death certificates established cause of death for individuals deceased after 31 December 2004. The average follow-up time was 5.2 years (range 0.1 to 5.9 years). A total of 102 patients (12%) died during follow-up, with cause of death obtained from death certificates and coded according to the International Classification of Diseases (ICD) standards. In addition to looking at overall mortality, the primary causes of death due to cancer (ICD9 140 to 239, ICD10 C00 to D48) and cardiovascular disease (CVD) (ICD9 401 to 459 or ICD10 I10 to I99) were examined.

### Statistical analysis

Results are expressed as median and (±) range, unless otherwise indicated, with p<0.05 indicating significance. Cohorts were compared using unpaired t-test and chi-square analysis for continuous and categorical variables respectively. As many values were not normally distributed, correlations between markers were calculated by Spearman's rank test. Differences in cumulative survival rates were compared between patients with varied MIC-1 levels. Exposure was computed from date of blood draw until date of death with censoring first for length of time interval of interest. Unadjusted and adjusted relative risks (RRs) of death and 95% CI were estimated by use of Cox proportional hazard models. Adjusted RRs were estimated after first fitting models with variables identified in previous analyses as independent risk factors. Survival curves were computed by the Kaplan-Meier method and compared among risk stratification groups using the log-rank statistic. Where correlation coefficients were compared, correlations r-value was determined by the correlation z test and compared using the Fisher r to z transformation. Comparison of relative risks was performed as previously described²⁵. Analyses were performed with StatView 5.0 software (SAS Inc., Campus Drive, Cary, NC, United States of America).

### Results and Discussion

### Characteristics of male control population cohort

Of the 876 subjects enrolled, 102 died during the follow-up time. Of these, 30 died of cancer and 46 patients suffered from cardiovascular events, of which 13 were myocardial ischaemic events. The remaining 26 patients died of other causes or could not be confidently classified on the basis of their death certificate (Table 5). The median serum MIC-1 level was 934 pg/ml (range 156-9638 pg/ml; interquartile range 628 pg/ml).

**Table 5 Descriptive data for control population cohort of 876 apparently healthy males**

| **Variable** | **Specific** | **Data¹** |
|---|---|---|
| Age at blood draw (years) | | 68±12 |
| Follow-up time (years) | | 5.3±0.5 |
| Smoking status n (%) | | |
| | Never | 330 (38%) |
| | Current or past | 513 (58%) |
| | Unknown | 33 (4%) |
| MIC-1 level (pg/ml) | | 935±627 |
| BMI (kg/m²) | | 25.9±3.9 |
| Mortality n (%) | | |
| | Alive | 774 (88%) |
| | Dead | 102(12%) |
| Cause of death n (%) | | |
| | Cardiovascular | 43 (42%) |
| | *Follow-up time* | *3.3*±*2.9* |
| | Cancer | 33 (33%) |
| | *Follow-up time* | *2.7*±*2.2* |
| | Other | 26 (25%) |
| | *Follow-up time* | *3.1*±*1.7* |

| | | |
|---|---|---|
| ¹Data is presented as median ± interquartile range or absolute number (% of cohort) | | |

### Serum MIC-1 level is a predictor of death in a normal male population

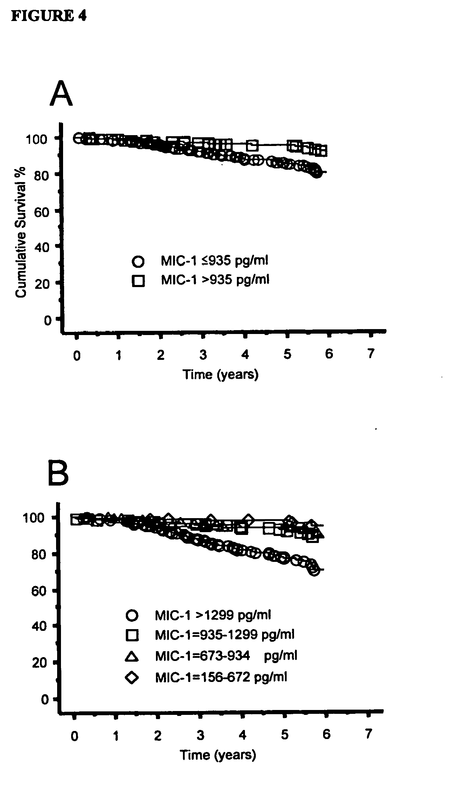

Serum MIC-1 levels were significantly correlated with age and predicted mortality in the all-male cohort with an age-adjusted relative risk of death of 3.38 (95%CI=1.38-8.26). Serum MIC-1 levels above the median (935+627 pg/ml) of the 876 subjects of the male control cohort were associated with death (p<0.0001). A Kaplan Meier plot of subjects stratified by the serum MIC-1 median (935 pg/ml) shows that subjects with MIC-1 levels greater than the median had a significantly poorer survival compared to survival for subjects with MIC-1 levels below the median (82% compared to 94%; p<0.0001; Figure 4A). Further, the serum MIC-1 level was significantly higher in subjects who ultimately died within the study period (median = 885 pg/ml for subjects that survived compared to median = 1432 pg/ml for subjects that died; p<0.0001). However, subjects who died were significantly older than those that survived (median age at blood draw for survivors = 67 years compared to median age at blood draw for those that died = 75 years; p<0.0001). Further, serum MIC-1 level correlated with age (p=0.458; p<0.0001). The cohort was divided into quartiles based on serum MIC-1 levels and re-examined as shown in Figure 4B. The majority of subjects that died within the follow-up period had serum MIC-1 levels in the top quartile (>1299 pg/ml). Further, serum MIC-1 level in the top quartile was significantly associated with mortality (p<0.0001), with only 74% of subjects in this quartile surviving compared to greater than 90% of subjects in the lower three quartiles. Men who ultimately died of non-cardiovascular or non-cancer causes, cardiovascular disease and cancer were all more likely to have serum MIC-1 levels in the highest quartile (p=0.0034, p<0.0001, p= 0.0429, respectively). Using the Cox proportional hazards model, a serum MIC-1 level in the top quartile engendered a more than 7 fold increased risk of death (RR 7.05; 95%CI 3.49-14.25) as shown in Table 6. When adjusted for other risks for mortality, history of smoking, BMI and age, a serum MIC-1 in the top quartile still was significantly related to risk of future mortality (RR 3.38; 95%CI 1.38-8.26; Table 6).

**Table 6 Multivariate Cox proportional hazard analysis of all cause mortality in male control population cohort**

| | **Adjustment** | **n** | **Hazard ratio** | **95%CI** | **p** |
|---|---|---|---|---|---|
| ***MIC-1 quartile*** | † | | | | |
| 156-672 pg/ml | | 219 | 1 | | |
| 673-934 pg/ml | | 219 | 1.94 | 0.87-4.35 | 0.1078 |
| 935-1299 pg/ml | | 219 | 2.25 | 1.02-4.94 | 0.0434 |
| > 1299 pg/ml | | 219 | 7.05 | 3.49-14.25 | <0.0001 |
| | | | | | |
| ***MIC-1 quartile*** | ‡ | | | | |
| 156-672 pg/ml | | 219 | 1 | | |
| 673-934 pg/ml | | 219 | 1.89 | 0.737-4.85 | 0.1854 |
| 935-1299 pg/ml | | 219 | 1.43 | 0.55-3.68 | 0.462 |
| > 1299 pg/ml | | 219 | 3.38 | 1.38-8.26 | 0.0077 |

| | | | | | |
|---|---|---|---|---|---|
| ^{†} Crude ^{‡} Adjusted for age, BMI and smoking history | | | | | |

Accordingly, serum MIC-1 level provides an independent and powerful predictor of future all cause mortality in a normal male population.

### Example 3 Association of serum MIC-1 in an independent cohort of twins

For validation purposes, the association of serum MIC-1 with survival was examined in an independent cohort of twins.

### Materials and Methods

### Twin cohort

The twin cohort included 308 subjects (comprising 154 same-sex twin pairs) nested within the Swedish Twin Registry²¹, currently the largest population-based twin registry in the world registering more than 85,000 twin pairs born since 1886. The subset of twins for the current analyses participated in studies of aging^{22,23}. Zygosity had been previously determined by asking pairs if they were "as similar as peas in a pod" or "no more alike than siblings in general"; and zygosity was confirmed for all pairs by either restriction fragment length polymorphism (RFLP) or serologic testing and microsatellite markers.

### Follow-up assessment of twin cohort

For the 308 subjects within the twin population, death dates were obtained through the Registry of the Total Population until the end of 2003 and causes of death were available through linkage with the Swedish Cause of Death Registry using each twin's personal registration number (PRN). The Cause of Death Registry, established in 1961, is 99% complete for all of the Swedish population who have died since 1961. Causes of death were updated until the end of 2001. Deaths from specific causes are obtained from death certificates and were coded according to the International Classification of Diseases (ICD) standards. In addition to examining overall mortality, the primary causes of death due to cancer (ICD9 140 to 239, ICD10 C00 to D48) and CVD (ICD9 401 to 459 or ICD10 I10 to I99) were evaluated. Information on age and sex were derived from the Swedish Twin Registry. Observation time for each twin was calculated from date of entry into the cohort, as defined by the date of blood draw (1992-1996), until the occurrence of death or censoring (survival) at the end of the observation period (31 March 2003).

### Determination of telomere length

Whole blood for telomere analysis was available for 154 twin pairs²⁴. Telomere length was assessed by terminal restriction fragment (TRF) analysis, which relies on restriction enzyme digestion and Southern blot hybridization of a minimum of 105 cells to measure the average length of telomeres. This was one of the first and most widely used techniques and produces reliable results, although it biases the results against the detection of short telomeres. Telomere length for study participants was measured in a series of 18 batches. In order to account for potential batch-specific differences in telomere measurements, telomere lengths from each respective batch were standardised separately to fit a normal distribution and then the standardised telomere lengths from each batch were pooled for the analysis of a continuous telomere length variable. When telomere length was analysed as a categorical variable, each batch was divided independently into quartiles based on length, and then each quartile was pooled across the batches. Both the standardisation and the quartile methods were measures that control for interbatch measurement variation. To verify controlling for between-batch variations, analyses were restricted to standardizsed telomere lengths of the 33 twin pairs where co-twins were measured in the same batch.

### Determination of serum MIC-1 levels

MIC-1 serum concentrations (pg/ml) were determined using a sensitive sandwich ELISA², established using the mouse monoclonal antibody (MAb) 26G6H6 for antigen capture and a sheep polyclonal antibody (PAb) 233B3-P for detection, as described above. All samples were assayed in triplicate and the coefficient of variation between samples was less than 12 percent.

### Statistical analysis

Statistical analysis was performed as for Example 2.

### Results and Discussion

### Characteristics of twin cohort

As shown in Table 7, the subjects in the twin cohort were significantly older than the subjects of male control population cohort at blood draw (median for the twin cohort = 78 years compared to median for the male control population cohort = 68 years; p<0.0001). The twin cohort had higher serum MIC-1 levels (median=1393 pg/ml; range 428-8064 pg/ml; interquartile range 1056 pg/ml; p<0.0001) than the male control population cohort, and serum MIC-1 level was significantly correlated with age (ρ=0.614; p<0.0001). Additionally, the twin population had a significantly lower BMI (median= 23.84 kg/m²) than the male control population cohort(median = 25.92 kg/m²; p<0.0001).

**Table 7 Descriptive data for twin cohort of 308 subjects**

| **Variable** | **Specific** | **Data¹** |
|---|---|---|
| Age at blood draw (years) | | 78±14 |
| Follow-up time (years) | | 9.4±7.7 |
| Sex, n (%) | | |
| | Male | 98 (32%) |
| | Female | 210 (68%) |
| Zygosity n (%) | | |
| | Monozygote | 168 (56%) |
| | Dizygote | 140 (44%) |
| Smoking status n (%) | | |
| | Never | 203 (67%) |
| | Current or past | 105 (33%) |
| | Unknown | 0 |
| MIC-1 level (pg/ml) | | 1393±1056 |
| BMI (kg/m²) | | 23.8±3.6 |
| Mortality n (%) | | |
| | Alive | 109 (35%) |
| | Dead | 199 (65%) |
| Cause of death n (%) | | |
| | Cardiovascular | 92 (46%) |
| | *Follow-up time* | *5.3*±*4.4* |
| | Cancer | 29 (15%) |
| | *Follow-up time* | *4.7*±*4.0* |
| | Other | 78 (39%) |
| | *Follow-up time* | *8.5*±*6.9* |
| ¹Data is presented as median ± interquartile range or absolute number (% of cohort) | | |

Of the 199 subjects that died, 29 died of cancer, and 92 of cardiovascular causes, of which 41 were myocardial infarcts. Subjects who ultimately died from the twin cohort were older at blood sampling than the subjects who died from the male control population cohort (median = 83 years for the twin cohort compared to median = 71 years for the male control population cohort; p<0.0001). In contrast to the male control population cohort, the twin cohort was more than 67% female, although there was no significant differences in serum MIC-1 levels between the sexes (median MIC-1 level for males in the twin cohort = 1407 pg/ml compared to median MIC-1 level for females in the twin cohort = 1383 pg/ml; p=0.5149). However, females in the twin cohort were significantly older than males in the twin cohort at blood sampling (median age for females in the twin cohort = 82 years compared to median age for males in the twin cohort = 74 years; p<0.0001). There was no difference in death rates between males and females (p=0.6268). Interestingly, serum MIC-1 was negatively correlated with telomere length (p=-0.181; p=0.0011). Serum IL-6 levels were available for 117 subjects from the twin cohort and CRP levels were available from 109 subjects from the twin cohort. Serum MIC-1 level was correlated with serum IL-6 (p=0.233; p=0.0121); however, serum MIC-1 was not correlated with the serum level of CRP (p=0.054; p=0.5765). As serum IL-6, CRP, age and telomere length and BMI are all established markers of mortality, their ability to predict mortality was compared to that of serum MIC-1 level.

### MIC-1 is a validated independent marker of future mortality

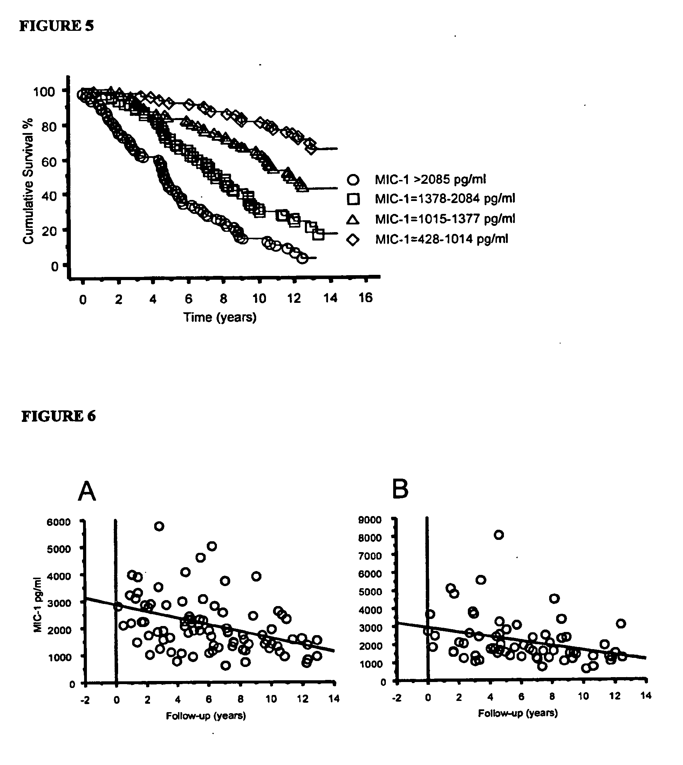

The serum MIC-1 levels of subjects in the twin cohort were stratified into quartiles. Serum MIC-1 predicted mortality, with increasing levels of serum MIC-1 associated with increased risk of mortality (p<0.0001; Table 8; Figure 5). In this cohort, only 6 % of subjects with serum MIC-1 level in the top quartile survived the follow-up period, compared to 69% of patients with serum MIC-1 levels in the lowest quartile.

Subjects that ultimately died of cancer, cardiovascular disease or other conditions were more likely to have had serum MIC-1 levels in the highest quartile (p = 0.0345, p < 0.0001, p = 0.0263, respectively). Subjects with serum MIC-1 levels in the top quartile had an increased risk of mortality (RR = 8.64; 95%CI = 5.41-13.78), confirming observations made in the all male control population cohort. However, in the twin cohort, any level of increase in MIC-1 serum level above the bottom quartile indicated an increased risk of death (Table 8). When adjustment was made for other factors associated with mortality (eg previous or current smoking history, BMI, sex, telomere length and age), serum MIC-1 levels in the top two quartiles remained independently associated with an increase risk of future mortality (top quartile: RR = 2.87, 95%CI = 1.68-4.91; second top quartile: RR = 1.99, 95%CI = 1.20-3.29; Table 8).

The twin cohort also validated the finding that serum MIC-1 is an independent predictor of mortality when further adjusted for telomere length, IL-6 and CRP. Only 108 subjects from the twin cohort had data available for both serum IL-6 and CRP levels. As the top two quartiles of serum MIC-1 significantly predicted mortality, when adjusted, serum MIC-1 was stratified according to the median (1392 pg/ml). In addition to adjusting for previous or current smoking history, BMI, sex, telomere length and age, hazard ratios were also adjusted for serum IL-6 and CRP levels. Serum MIC-1 level above the median, when adjusted for previous or current smoking history, BMI, sex, telomere length and age, serum IL-6 and serum CRP levels, was an independent predictor of mortality (RR=2.26; 95%CI=1.19-4.29; Table 8).

In the all male control population cohort and twin cohort, serum MIC-1 is not strongly associated with BMI (data not shown). This is likely due to relatively lower serum MIC-1 levels in these cohorts compared with disease specific populations (aside from cardiovascular disease populations). These results indicate that the serum MIC-1 levels that affect BMI are significantly higher in diseased populations²⁷. It has previously been shown that in heart failure patients, serum MIC-1 levels that affect BMI are likely to be greater than 3700 pg/ml²⁸. However, BMI was significantly higher and serum MIC-1 levels were lower in the younger all male population control cohort compared to the older twin cohort, indicating an inverse correlation of serum MIC-1 with BMI as previously described²⁷. Additionally, upon combining patients with serum MIC-1 levels greater than 3800 pg/ml in both the male control population and twin cohorts, serum MIC-1 trended towards being negatively associated with BMI (ρ=-0.351; p=0.0547). Patients with prostate cancer only have a significant relationship with BMI when MIC-1 levels are greater than 6000 pg/ml²⁷ and a similar relationship occurs in chronic renal disease (Breit *et al.* submitted to The Lancet).

Accordingly, serum MIC-1 levels have been validated to be an independent and powerful predictor of future all cause mortality in a population of twins that was predominantly female, with serum MIC-1 correlated with time to death in the twins cohort. As previously published, serum MIC-1 levels were correlated with age and other markers of mortality and ageing, specifically, IL-6 and CRP²⁶. Serum MIC-1 level is weakly but significantly correlated with telomere length, which may be influenced by a number of environmental variables. Oxidative stress significantly shortens telomere length and induces DNA damage potentially leading to replicative senescence (Breit *et al.* submitted to The Lancet), a marker of biological ageing.

**Table 8 Multivariate Cox proportional hazard analysis of all cause mortality in twin cohort**

| | **Adjustment** | **n** | **Hazard ratio** | **95%CI** | **p** |
|---|---|---|---|---|---|
| ***MIC-1 quartile*** | † | | | | |
| 428-1014 pg/ml | | 77 | 1 | | |
| 1015-1377 pg/ml | | 77 | 2.18 | 1.32-3.59 | 0.0023 |
| 1378-2084 pg/ml | | 77 | 4.25 | 2.64-6.85 | <0.0001 |
| >2085 pg/ml | | 77 | 8.92 | 5.55-14.33 | <0.0001 |
| | | | | | |
| ***MIC-1 quartile*** | ‡ | | | | |
| 428-1014 pg/ml | | 77 | 1 | | |
| 1015-1377 pg/ml | | 77 | 1.51 | 0.91-2.50 | 0.1093 |
| 1378-2084 pg/ml | | 77 | 2.09 | 1.25-3.47 | 0.0046 |
| >2085 pg/ml | | 77 | 3 | 1.74-5.16 | <0.0001 |
| | | | | | |
| ***MIC-1 quartile*** | * | | | | |
| 428-1014 pg/ml | | 23 | 1 | | |
| 1015-1377 pg/ml | | 21 | 1.21 | 0.48-3.06 | 0.6919 |
| 1378-2084 pg/ml | | 30 | 2.61 | 1.04-6.56 | 0.0418 |
| >2085 pg/ml | | 34 | 2.5 | 0.94-6.69 | 0.0675 |
| | | | | | |
| ***MIC-1 Haptile*** | * | | | | |
| 428-1392 pg/ml | | 44 | 1 | | |
| >1392 pg/ml | | 64 | 2.26 | 1.19-4.29 | 0.0125 |

| | | | | | |
|---|---|---|---|---|---|
| ^{†}Crude. ^{‡}Adjusted for age, sex BMI and smoking history and telomere length. *Adjusted for age, sex BMI and smoking history, telomere length, IL-6 and CRP. | | | | | |

### Serum MIC-1 levels predict mortality rate independently of genetic background

Despite being correlated with potential markers of biological ageing, of which a significant number predict mortality²⁶, the results indicate that serum MIC-1 level independently predicts mortality and is not influenced significantly by genetic background, as serum MIC-1 level was directly correlated with survival time and not influenced by twin zygosity. Where both members of a twin pair died, serum MIC-1 level was significantly and inversely correlated to survival time (r=0.344; p<0.0001). As shown in Figures 6A and 6B, there was no significant difference in the strength of the correlation between monozygotic (MZ) and dizygotic (DZ) twin pairs (MZ: r=0.419, p<0.0001; DZ: r=0.342, p=0.0046; Difference z=-0.51; p=0.2946, one tailed, p=0.5892 two tailed; Fisher r to z transformation). Additionally, using the Cox proportional hazards model there was no significant difference in the risk of death between MZ and DZ twins who had serum MIC-1 levels greater than the median at study entry (MZ: RR= 1.71, 95%CI = 1.06-2.77; DZ: RR= 2.17, 95%CI = 1.32-3.56), and these correlations were not significantly different (ratio of relative risk=1.27; 95%CI=0.63-2.53). Thus, serum MIC-1 levels had similar predictive power for mortality in monozygotic and dizygotic twins indicating that changes in serum MIC-1 relate to active disease processes rather than genetic background.

Accordingly, serum MIC-1 level is an important biomarker capable of predicting increased risk of all cause mortality.

### Example 4 MIC-1 serum concentration in prostate cancer patients - for illustration only

To verify the serum levels of MIC-1 in prostate cancer patients described in Example 1, a cohort of men diagnosed with prostate cancer was examined from the same study as described in Example 1, except that in this case, the cohort was larger and was followed for an additional 10 months.

### Materials and Methods

### Study cohort

This study used serum samples from 1442 prostate cancer subjects (from Cancer Prostate in Sweden (CAPS)) for the measurement of levels of MIC-1. Based on self-reported treatment history, samples were categorised as either pre-treatment (n = 431) or post-treatment (n = 1011).

### Follow-up assessment

With the use of each subject's unique national registration number, vital status was assessed from date of blood draw up until 15 January 2008, through record linkage to the Swedish Population Registry, and prostate cancer specific survival was obtained through linkage with the Cause of Death Registry up to 31 December 31 2005. Review of death certificates, performed by an oncologist, established cause of death for individuals deceased after 31 December 2005.

### Determination of MIC-1 serum levels

MIC-1 serum concentrations (pg/ml) were determined as described in Example 1. All samples were assayed in triplicate and the coefficient of variation between the samples was less than 12 percent.

### Statistical analysis

Differences in MIC-1 serum levels between clinical characteristics were tested using the Kruskal-Wallis test. Time-to-event analysis using death from prostate cancer as outcome was performed. Survival time was censored at time of death for subjects dying from causes other than prostate cancer. The association between MIC-1 serum level and prostate cancer death was assessed using Cox regression analysis with serum levels categorised into four groups based on quartiles of the distribution of MIC-1 levels among all patients, with the lowest category (ie the lower quartile) used as reference group. In analysis stratified by prognostic risk group, Cox regression analysis of log-transformed MIC-1 levels was performed. To evaluate the discriminatory power of MIC-1 serum levels on prostate cancer mortality, the concordance probability based on the parameter estimate from a Cox regression model was estimated³¹. The concordance estimate ranged between 0.5 and 1.0, with 1.0 representing perfect concordance between the prognostic variable and survival time.

The presence of competing risks were acknowledged using the cmprsk Package for the R programming language³² to estimate cumulative incidence of prostate cancer mortality. Gray's test³³ to assess differences in cumulative incidence between patients categorized according to quartiles of the distribution of MIC-1 levels was used. All P values reported were based on two-sided hypothesis.

### Results

### MIC-1 serum levels and clinical characteristics

Table 9 shows MIC-1 serum levels by clinical characteristics of patients. MIC-1 serum levels were significantly elevated across increasing level of T stage (P < 0.0001), M stage (P < 0.0001), Gleason sum (P < 0.0001), and diagnostic PSA level (P < 0.0001). No significant difference in MIC-1 serum levels between nodal negative and nodal positive patients was observed.

**Table 9 MIC-1 serum levels in larger prostate cancer cohort**

| | Patients | | MIC-1 serum level (pg/ml) | | |
|---|---|---|---|---|---|
| Characteristic | No. | (%) | Median | Range | P value¹ |
| Tumor stage | | | | | |
| T1 | 518 | (35.9) | 872 | 219-5090 | |
| T2 | 473 | (32.8) | 1008 | 176-6410 | |
| T3 | 373 | (25.9) | 1143 | 196-31252 | |
| T4 | 51 | (3.5) | 1276 | 143-9243 | |
| Tx | 27 | (1.9) | 961 | 236-8876 | <0.0001 |

| Nodal stage | | | | | |
|---|---|---|---|---|---|
| N0/Nx | 1394 | (96.7) | 1002 | 143-31252 | |
| N1 | 48 | (3.3) | 1094 | 356-9243 | 0.31 |

| Metastasis stage | | | | | |
|---|---|---|---|---|---|
| M0/Mx | 1302 | (90.3) | 974 | 176-12004 | |
| M1 | 140 | (9.7) | 1324 | 143-31252 | <0.0001 |

| Biopsy Gleason score | | | | | |
|---|---|---|---|---|---|
| 2-6 | 707 | (49.0) | 898 | 176-8876 | |
| 7 | 460 | (31.9) | 1100 | 234-12004 | |
| 8-10 | 244 | (16.9) | 1099 | 143-31252 | |
| Missing | 31 | (2.1) | 1040 | 219-5374 | <0.0001 |

| PSA level | | | | | |
|---|---|---|---|---|---|
| <20 ng/ml | 870 | (60.3) | 888 | 176-8876 | |
| 20-49 ng/ml | 237 | (16.4) | 1103 | 256-9243 | |
| >50 ng/ml | 296 | (20.5) | 1276 | 143-31252 | |
| Missing | 39 | (2.7) | 914 | 236-5259 | <0.0001 |

| | | | | | |
|---|---|---|---|---|---|
| ¹ Kruskal-Wallis test | | | | | |

### MIC-1 serum levels and prostate cancer death

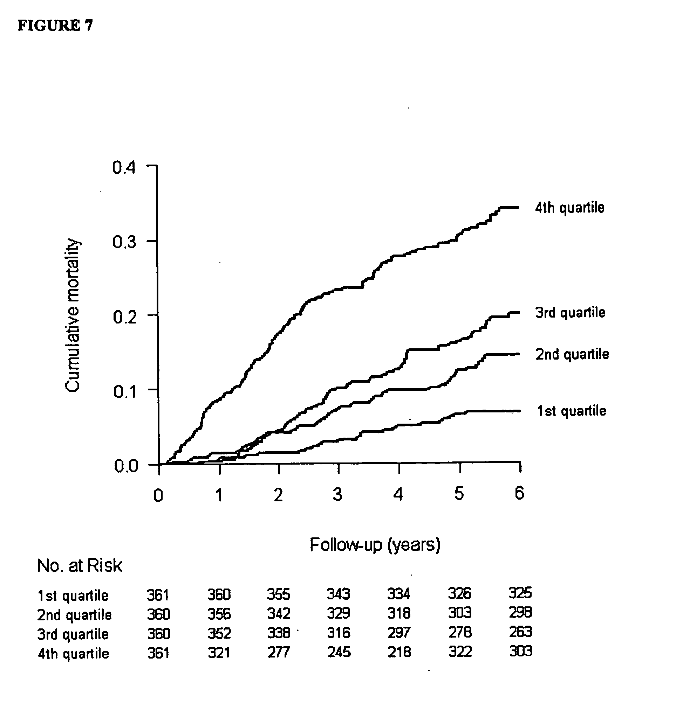

Overall, 380 (26%) of the 1442 men died during the follow-up and of those 265 (18%) had prostate cancer classified as their underlying cause of death. The average follow-up time was 4.9 years (range 0.1 to 6.8 years). The cohort was stratified into quartiles according to MIC-1 serum level. The distribution of MIC-1 serum levels in patients who ultimately died of prostate cancer was skewed toward the top quartile compared to surviving patients. As shown in Figure 7 and Table 10, after six years of follow-up, the cumulative incidence of death from prostate cancer was 7% among subjects with MIC-1 serum concentrations below 710 pg/ml (ie in the bottom quartile, referred to as the 1st quartile in Figure 7) and 34% among subjects with MIC-1 serum concentrations above 1456 pg/ml (ie in the top quartile, referred to as the 4th quartile in Figure 7) (P < 0.0001), corresponding to a six-fold relative risk (hazard ratio [HR], 6.33; 95% confidence interval [CI], 4.11-9.74; Table 10). In multivariate analysis that adjusted for the effects of the established prognostic factors Gleason sum, TNM stage, and diagnostic PSA level, higher MIC-1 levels remained associated with prostate cancer death (adjusted HR, 3.58; 95 % CI, 2.28-5.63; Table 10).

**Table 10 Risk of death from prostate cancer among 1442 prostate cancer patients**

| MIC-1 level (pg/ml) | No. of patients | No. of prostate cancer deaths | Crude HR | (95% CI) | Adjusted HR* | (95% CI) |
|---|---|---|---|---|---|---|
| *All samples* | | | | | | |
| <710 | 361 | 25 | 1.00 | | 1.00 | |
| 710-1006 | 360 | 51 | 2.10 | (1.30 to 3.39) | 1.48 | (0.91 to 2.42) |
| 1006-1456 | 360 | 68 | 2.90 | (1.83 to 4.59) | 1.75 | (1.09 to 2.81) |
| >1456 | 361 | 121 | 6.33 | (4.11 to 9.74) | 3.58 | (2.28 to 5.63) |
| P trend | | | <0.0001 | | <0.0001 | |

| *Pretreatment samples* | | | | | | |
|---|---|---|---|---|---|---|
| <710 | 112 | 2 | 1.00 | | 1.00 | |
| 710-1006 | 108 | 6 | 3.12 | (0.63 to 15.47) | 2.20 | (0.44 to 11.04) |
| 1006-1456 | 105 | 10 | 5.49 | (1.2 to 25.04) | 3.15 | (0.65 to 15.18) |
| >1456 | 106 | 20 | 12.08 | (2.82 to 51.70) | 9.61 | (2.22 to 41.57) |
| P trend | | | <0.0001 | | <0.0001 | |

| *Posttreatment samples* | | | | | | |
|---|---|---|---|---|---|---|
| <710 | 249 | 23 | 1.00 | | 1.00 | |
| 710-1006 | 252 | 45 | 2.00 | (1.21 to 3.30) | 1.40 | (0.84 to 2.36) |
| 1006-1456 | 255 | 58 | 2.66 | (1.64 to 4.31) | 1.61 | (0.98 to 2.65) |
| >1456 | 255 | 101 | 5.95 | (3.78 to 9.37) | 3.09 | (1.91 to 5.00) |
| P trend | | | <0.0001 | | <0.0001 | |

| | | | | | | |
|---|---|---|---|---|---|---|
| * Hazard ratios from a multiple Cox model including serum MIC-1 levels, clinical T stage, biopsy Gleason score, diagnostic serum PSA level, and metastatic status as covariates | | | | | | |

Separate assessment of MIC-1 serum levels was performed among men with blood drawn pre-treatment (n = 431) and post-treatment (n = 1,011). Compared with the total study cohort, an even stronger association between pre-treatment MIC-1 serum levels and prostate cancer survival was observed (Table 10). Subjects within the top quartile (ie with serum MIC-1 level of >1456 pg/ml) had a more than twelve-fold higher death rate than those in the bottom quartile (iw with MIC-1 levels <710 pg/ml, HR, 12.08; 95% CI, 2.82-51.70). In adjusted analysis, pre-treatment MIC-1 levels remained an independent prognostic factor with an almost ten-fold higher death rate in the top quartile compared with the bottom quartile (HR, 9.61; 95% CI, 2.22-41.57). Subjects with post-treatment MIC-1 serum levels in the top quartile were also associated with increased risk of prostate cancer death with an almost six-fold higher death rate in compared to that in the bottom quartile (HR, 5.95; 95% CI, 3.78-9.37; Table 10). Adjustment for Gleason sum, TNM stage, and diagnostic PSA level attenuated the strength of association between post-treatment MIC-1 serum levels and prostate cancer death; however, post-treatment serum MIC-1 levels in the top quartile (ie >1456 pg/ml) remained an independent predictor of prognosis with a three-fold higher cancer death rate in the highest compared with the lowest category (HR, 3.09; 95% CI, 1.91-5.00; Table 10).

### MIC-1 serum levels in subjects with clinically localised disease

Analysis was then restricted to subjects with clinically localised disease (ie subjects with a T score of T1/T2 and an N score of N0/Nx and a M score of M0/Mx) because individual prognostication and management is a special challenge among these subjects. To explore the prognostic value of MIC-1 serum levels in more homogeneous sub-groups, subjects were further stratified into the traditional low risk (PSA<10 and Gleason sum<7), intermediate risk (PSA of 10 to 20 or Gleason sum of 7), and high risk (PSA>20 and Gleason sum>7) groups. However, since only one subject died from prostate cancer during follow-up in the low risk group, the low and intermediate risk groups were pooled into one risk group. Cox regression analysis of log-transformed MIC-1 serum levels revealed a significant association with prostate cancer death among men in the low/intermediate risk group as well as in the high risk group (P = 0.0001 and P = 0.001, respectively; Table 11). The concordance probability estimate, assessing the predictive strength of the Cox model, among men in the low/intermediate risk group was 0.71 (SE, 0.04) while men in the high risk group had a concordance probability of 0.66 (SE, 0.04).

Analysis restricted to samples being drawn pre- or posttreatment revealed a significant association between log-transformed MIC-1 serum levels and prostate cancer death both among men in the low/intermediate risk group (pretreatment, P = 0.009; post-treatment, P = 0.006) and among med in the high risk group (pretreatment, P = 0.02; posttreatment, P = 0.01). Both among men with pre- and post-treatment blood draw, estimated concordance probabilities were higher among men with low/intermediate risk as compared to med in the high risk group (0.72 vs. 0.69; and 0.70 vs. 0.65, respectively; Table 3).

**Table 11 Risk of Death from prostate cancer among 857 subjects with localised disease**

| MIC-1 level | No. of patients | No. of prostate cancer deaths | HR | (95% CI) | P value | Concordance probability (SE) |
|---|---|---|---|---|---|---|
| *All samples* | | | | | | |
| Low/intermediate risk | 632 | 12 | 6.34 | (2.46 to 16.29) | 0.0001 | 0.71 (0.04) |
| High risk | 225 | 29 | 3.07 | (1.57 to 5.98) | 0.001 | 0.66 (0.04) |

| *Pretreatment samples* | | | | | | |
|---|---|---|---|---|---|---|
| Low/intermediate risk | 256 | 6 | 7.00 | (1.64 to 29.93) | 0.009 | 0.72 (0.06) |
| High risk | 76 | 7 | 4.16 | (1.25 to 13.88) | 0.02 | 0.69 (0.06) |

| *Posttreatment samples* | | | | | | |
|---|---|---|---|---|---|---|
| Low/intermediate risk | 376 | 6 | 5.84 | (1.64 to 20.8) | 0.006 | 0.70 (0.05) |
| High risk | 149 | 22 | 2.72 | (1.22 to 6.07) | 0.01 | 0.65 (0.05) |

| | | | | | | |
|---|---|---|---|---|---|---|
| * The prognostic role of MIC-1 serum level is tested within each prognostic risk group category. Log-transformed MIC-1 serum level was modeled as a continuous variable. | | | | | | |

This example confirms the association between MIC-1 serum concentrations and disease stage, and additionally demonstrates, for the first time, the prognostic value of serum MIC-1 level as a marker to discriminate between fatal and non-fatal prostate cancer. In multivariate analysis, adjustment for the established prognostic factors Gleason sum, clinical stage and diagnostic PSA level, did not materially affect the independent prognostic value of MIC-1. Importantly, in organ-confined disease, an elevated serum MIC-1 level was an independent predictor of fatal prostate cancer. The results therefore indicate that serum MIC-1 levels can prognose prostate cancer death and disease progression.

Due to the impact of screening for prostate cancer with PSA, prostate cancer is increasingly diagnosed at a localised stage. Since progression-free survival in subjects with localised disease managed with watchful waiting is high^{13,14} and disease outcome cannot be accurately predicted, over treatment of subjects with low risk disease is common. Management by active surveillance with selective delayed intervention based on early PSA changes has been proposed as a strategy to reduce over treatment of subjects with indolent disease. However, although both baseline PSA measurements and rate of PSA change are important prognostic factors, they perform poorly in distinguishing those who will develop a fatal prostate cancer from those at low risk of disease progression.¹⁶ The results obtained in this example show that both pre-treatment and post-treatment serum MIC-1 levels can be used to predict disease outcome in subjects with organ-confined disease. Therefore, a high MIC-1 concentration at diagnosis may identify subjects that would benefit from early systemic adjuvant treatment in addition to local treatment.

In summary, with the use of serum MIC-1 concentrations, prostate cancer subjects were stratified into groups with substantially different prostate cancer mortality rates independent of traditional prognostic markers of disease. There was an association between both pre-treatment and post-treatment serum MIC-1 levels and clinical outcome in subjects with clinically localised high risk disease, a group whose prognosis is difficult to assess. Additionally, serum MIC-1 levels identified subjects with low to intermediate risk disease who ultimately progressed. Further, evaluation of MIC-1 stromal staining in addition to serum MIC-1 level determination may allow additional discriminatory capacity between fatal and non fatal localised prostate cancer.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed in Australia or elsewhere before the priority date of each claim of this application.

### REFERENCES

1. Bootcov MR, et al. (1997) MIC-1, a novel macrophage inhibitory cytokine, is a divergent member of the TGF-beta superfamily. Proc Natl Acad Sci USA 94(21): 11514-11519.
2. Brown DA., et al. (2002) Antibody-based approach to high volume genotyping for MIC-1 polymorphism. Biotechniques;33(1):118-120, 22, 24 passim.
3. Moore AG., et al. (2000) The transforming growth factor-β superfamily cytokine macrophage inhibitory cytokine-1 is present in high concentrations in the serum of pregnant women. J Clin Endocrinol Metab; 85(12): 4781-4788.
4. Bottner M., et al. (1999) Expression of a novel member of the TGF-beta superfamily, growth/differentiation factor-15/macrophage-inhibiting cytokine-1 (GDF-15/MIC-1) in adult rat tissues. Cell Tissue Res 297(1): 103-110.
5. Fairlie WD., et al. (1999) MIC-1 is a novel TGF-beta superfamily cytokine associated with macrophage activation. J Leukoc Biol 65(1): 2-5.
6. Bauskin AR., et al. (2006) Role of macrophage inhibitory cytokine-1 in tumourigenesis and diagnosis of cancer. Cancer Res 66(10): 4983-4986.
7. Welsh JB., et al. (2003) Large-scale delineation of secreted protein biomarkers overexpressed in cancer tissue and serum. Proc Natl Acad Sci U S A 100(6): 3410-3415.
8. Brown DA., et al. (2006) Measurement of serum levels of macrophage inhibitory cytokine 1 combined with prostate-specific antigen improves prostate cancer diagnosis. Clin Cancer Res 12(1): 89-96.
9. Li PX., et al., (2000) Placental transforming growth factor-beta is a downstream mediator of the growth arrest and apoptotic response of tumour cells to DNA damage and p53 overexpression. J Biol Chem 275(26): 20127-20135.
10. Kannan K., et al. (2000) Profile of gene expression regulated by induced p53; connection to the TGF-beta family. FEBS Lett 470(1). 77-82.
11. Tan M., et al., (2000) PTGF-beta, a type beta transforming growth factor (TGF-beta) superfamily member, is a p53 target gene that inhibits tumour cell growth via TGF-beta signalling pathway. Proc Natl Acad Sci USA 97(1): 109-114.
12. Schraudenbach P. and Bermejo CE. (2007) Management of the complications of radical prostatectomy. Curr Urol Rep 8(3):197-202.
13. Johansson JE., et al. (2004) Natural history of early, localized prostate cancer. Jama 291(22): 2713-2719.
14. Albertsen PC., et al. (2005) 20-year outcomes following conservative management of clinically localized prostate cancer. Jama; 293(17): 2095-2101.
15. Bill-Axelson A., et al. (2005) Radical prostatectomy versus watchful waiting in early prostate cancer. N Engl J Med 352(19): 1977-1984.
16. Fall K., et al. (2007) Prostate-specific antigen levels as a predictor of lethal prostate cancer. J Natl Cancer Inst;99(7): 526-532.
17. Albertoni M., et al. (2002) Anoxia induces macrophage inhibitory cytokine-1 (MIC-1) in glioblastoma cells independently of p53 and HIF-1. Oncogene;21(27): 4212-4219.
18. Baek SJ., et al. (2001) Cyclooxygenase inhibitors regulate the expression of a TGF-beta superfamily member that has proapoptotic and antitumourigenic activities. Mol Pharmacol 59(4): 901-908.
19. Ferrari N., et al. (2005) The transforming growth factor-beta family members bone morphogenetic protein-2 and macrophage inhibitory cytokine-1 as mediators of the antiangiogenic activity of N-(4-hydroxyphenyl)retinamide. Clin Cancer Res. 11(12): 4610-4619.
20. Lee DH. et al. (2003) Macrophage inhibitory cytokine-1 induces the invasiveness of gastric cancer cells by up-regulating the urokinase-type plasminogen activator system. Cancer Res. 63(15): 4648-4655.
21. Lichtenstein, P. et al. (2002) The Swedish Twin Registry: a unique resource for clinical, epidemiological and genetic studies. J. Intern. Med. 252, 184-205.
22. McCleam, G. E. et al. (1997) Substantial genetic influence on cognitive abilities in twins 80 or more years old. Science 276, 1560-1563.
23. Finkel, D. et al. (2005) The longitudinal relationship between processing speed and cognitive ability: genetic and environmental influences. Behav Genet 35, 535-549.
24. Bakaysa, S. L. et al. (2007) Telomere length predicts survival independent of genetic influences. Aging Cell 6, 769-774.
25. Altman, D. G. & Bland, J. M. (2003) Interaction revisited: the difference between two estimates. BMJ 326, 219
26. Simm, A. et al. (2008) A. Potential biomarkers of ageing. Biol. Chem. 389, 257-265
27. Johnen, H. et al. (2007). Tumor-induced anorexia and weight loss are mediated by the TGF-beta superfamily cytokine MIC-1. Nat. Med. 13, 1333-1340
28. Kempf, T et al. (2007) Prognostic utility of growth differentiation factor-15 in patients with chronic heart failure. J. Am. Coll. Cardiol. 50, 1054-1060
29. Brown, DA et al. (2003) MIC-1 serum level and genotype: associations with progress and prognosis of colorectal carcinoma. Clin Cancer Res 9:2642-2650.
30. Bauskin et al. (2005) The propeptide mediates formation of stomal stores of PROMIC-1: Role in Determining Prostate Cancer Outcome. Cancer Res 65(6) 2330-2336
31. Gonen, M. and Heller, G. (2005) Concordance probability and discriminative power of proportional hazards regression. Biometrika 92: 965-970
32. Gray RJ. (2001) cmprsk Package [serial on line] Boston: Department of Biostatistical Science, Dana-Farber Cancer Institute. Accessed at http://biowww.dfci.harvard.edu/~gray on 17 October 2008
33. Gray, R. J. (1988) A class of K-sample tests for comparing the cumulative incidence of a competing risk. Ann Stat, 16: 1141-1154, 1988
34. Sobin, L.H. and Wittekind, Ch. (eds) (2002) TNM Classification of Malignant Tumours, 6th edition. John Wiley & Sons, Hoboken, New Jersey, United States of America
35. WO 01/81928

## Claims

1. A method of prognosis of overall survival of an apparently healthy subject, the method comprising detecting an elevated amount of MIC-1 in a test body sample from said subject, wherein the elevated amount of MIC-1 is associated with an increased likelihood of death of the subject.

2. The method of claim 1, wherein the elevated amount of MIC-1 in a test body sample predicts an increased likelihood of death from any cause other than accident or misadventure.

3. The method of claim 1 or 2, wherein the elevated amount of MIC-1 predicts an increased likelihood of death of the subject within a period of 10 years of taking the test body sample.

4. The method of any one of claims 1 to 3, wherein the test body sample is a serum sample.

5. The method of any one of claims 1 to 4, wherein the method comprises detecting an elevated amount of MIC-1 that is > 1.3 ng/mL.

6. The method of any one of claims 1 to 5, wherein the elevated amount of MIC-1 in the test body sample is detected by:
(i) determining the amount of MIC-1 present in the said test body sample; and
(ii) comparing said amount of MIC-1 against an amount or a range of amounts of MIC-1 present in comparative body sample(s) taken from normal subject(s).

7. The method of claim 6, wherein the normal subject(s) are age-matched within 5 years of the age of the subject from which the relevant test body sample has been taken.

8. The method of any one of claims I to 7, wherein the elevated amount of MIC-1 in a test body sample is an increase in the amount of MIC-1 within a subject detected using serial measurement by:
(i) determining the amount of MIC-1 present in the said test body sample; and
(ii) comparing said amount of MIC-1 against an amount or a range of amounts of MIC-1 present in comparative body sample(s) taken from the same subject at an earlier time point.

9. The method of any one of claims 6 to 8, wherein the method comprises detecting an increase in the amount of MIC-1 of>0.6 ng/ml.

10. The method of any one of claims 1 to 9, wherein the subject is more than 35 years of age.

## Patentansprüche

1. Verfahren zur Prognose des Gesamtüberlebens eines offensichtlich gesunden Individuums, wobei das Verfahren das Nachweisen einer erhöhten Menge MIC-1 in einer Testkörperprobe aus dem Individuum umfasst, wobei die erhöhte Menge MIC-1 mit einer erhöhten Wahrscheinlichkeit für Tod des Individuums einhergeht.

2. Verfahren nach Anspruch 1, wobei die erhöhte Menge MIC-1 in einer Testkörperprobe eine erhöhte Wahrscheinlichkeit für Tod aus einem anderen Grunde als Unfall oder Missgeschick vorhersagt.

3. Verfahren nach Anspruch 1 oder 2, wobei die erhöhte Menge MIC-1 eine erhöhte Wahrscheinlichkeit für Tod des Individuums in einem Zeitraum von 10 Jahren der Entnahme der Testkörperprobe vorhersagt.

4. Verfahren nach Anspruch 1 bis 3, wobei die Testkörperprobe eine Serumprobe ist.

5. Verfahren nach einem der Ansprüche 1 bis 4, wobei das Verfahren das Nachweisen einer erhöhten Menge MIC-1 umfasst, die >1,3 ng/ml ist.

6. Verfahren nach einem der Ansprüche 1 bis 5, wobei die erhöhte Menge MIC-1 in der Testkörperprobe nachgewiesen wird durch:
(i) Bestimmen der in der Testkörperprobe vorhandenen Menge MIC-1; und
(ii) Vergleichen der Menge MIC-1 gegen eine Menge oder einen Bereich von Mengen MIC-1, die in einer oder mehreren Vergleichskörperprobe(n) zugegen ist/sind, die aus einem oder mehreren normalen Individuum/Individuen entnommen wurde(n).

7. Verfahren nach Anspruch 6, wobei das normale Individuum oder die normalen Individuen innerhalb von 5 Jahren mit dem Alter des Individuums übereinstimmen, aus dem die relevante Testkörperprobe entnommen wurde.

8. Verfahren nach einem der Ansprüche 1 bis 7, wobei die erhöhte Menge MIC-1 in einer Testkörperprobe ein Anstieg der Menge MIC-1 in einem Individuum ist, der nachgewiesen wird mittels serieller Messung durch:
(i) Bestimmen der in der Testkörperprobe vorhandenen Menge MIC-1; und
(ii) Vergleichen der Menge MIC-1 gegen eine Menge oder einen Bereich von Mengen MIC-1, die in einer oder mehreren Vergleichskörperprobe(n) zugegen ist/sind, die aus dem gleichen Individuum zu einem früheren Zeitpunkt entnommen wurde(n).

9. Verfahren nach einem der Ansprüche 6 bis 8, wobei das Verfahren das Nachweisen eines Anstiegs der Menge MIC-1 von >0,6 ng/ml umfasst.

10. Verfahren nach einem der Ansprüche 1 bis 9, wobei das Individuum älter als 35 Jahre ist.

## Revendications

1. Procédé de pronostic de la survie globale d'un sujet apparemment sain, le procédé comprenant la détection d'une quantité élevée de MIC-1 dans un échantillon corporel d'essai dudit sujet, dans lequel la quantité élevée de MIC-1 est associée à une probabilité augmentée de décès du sujet.

2. Procédé de la revendication 1, dans lequel la quantité élevée de MIC-1 dans un échantillon corporel d'essai prédit une probabilité accrue de décès dû à une cause quelconque autre que l'accident ou désagrément.

3. Procédé de la revendication 1 ou 2, dans lequel la quantité élevée de MIC-1 prédit une probabilité accrue de décès du sujet dans une période de 10 ans suivant le prélèvement de l'échantillon corporel d'essai.

4. Procédé de l'une quelconque des revendications 1 à 3, dans lequel l'échantillon corporel d'essai est un échantillon de sérum.

5. Procédé de l'une quelconque des revendications 1 à 4, dans lequel le procédé comprend la détection d'une quantité élevée de MIC-1 qui est > 1,3 ng/ml.

6. Procédé de l'une quelconque des revendications 1 à 5, dans lequel la quantité élevée de MIC-1 dans l'échantillon corporel d'essai est détectée par :
(i) détermination de la quantité de MIC-1 présente dans ledit échantillon corporel d'essai ; et
(ii) comparaison de ladite quantité de MIC-1 à une quantité ou une plage de quantités de MIC-1 présentes dans un/des échantillon(s) corporel(s) comparatif(s) prélevé(s) sur des sujet(s) normal/normaux.

7. Procédé de la revendication 6, dans lequel le(s) sujet(s) normal/normaux sont associés par âge à au plus 5 ans de l'âge du sujet à partir duquel l'échantillon corporel d'essai a été prélevé.

8. Procédé de l'une quelconque des revendications 1 à 7, dans lequel la quantité élevée de MIC-1 dans un échantillon corporel d'essai est une augmentation de la quantité de MIC-1 chez un sujet détectée en utilisant une mesure sérielle par :
(i) détermination de la quantité de MIC-1 présente dans ledit échantillon corporel d'essai ; et
(ii) comparaison de ladite quantité de MIC-1 à une quantité ou une plage de quantités de MIC-1 présentes dans un/des échantillon(s) corporel(s) comparatif(s) prélevé(s) sur le même sujet à un temps précédent.

9. Procédé de l'une quelconque des revendications 6 à 8, le procédé comprenant la détection d'une augmentation de la quantité de MIC-1 de > 0,6 ng/ml.

10. Procédé de l'une quelconque des revendications 1 à 9, dans lequel le sujet est âgé de plus de 35 ans.
